# EUROPEAN PATENT APPLICATION

(11) **EP 4 140 501 A1**
(43) Date of publication of application: **01.03.2023**
(21) Application number: 21793632.7
(22) Date of filing: 21.04.2021
(51) Int. Cl.: A61K 39/00, A61K 39/395, A61P 31/00, A61P 35/00, C07K 14/705, C07K 16/28, C07K 19/00, A61K 47/64, A61K 47/68, C12N 15/13

(54) **IMPROVED PEPTIDE VACCINE**

(30) Priority: 23.04.2020 JP 2020076785
(71) Applicant: BrightPath Biotherapeutics Co., Ltd., Kawasaki-shi, Kanagawa 210-0821 (JP); Kagoshima University, Kagoshima-shi, Kagoshima 890-8580 (JP)
(72) Inventor: SASAKURA Yukie, Yokohama-shi, Kanagawa 230-0052 (JP); NAKAMURA Norihiro, Kawasaki-shi, Kanagawa 210-0821 (JP); MISHIMA Yuji, Kawasaki-shi, Kanagawa 210-0821 (JP); MATSUMOTO Noriko, Kawasaki-shi, Kanagawa 210-0821 (JP); ISODA Fumiko, Kawasaki-shi, Kanagawa 210-0821 (JP); ITO Yuji, Kagoshima-shi, Kagoshima 890-0065 (JP)
(74) Representative: Plasseraud IP
(86) International application number: PCT/JP2021/016133
(87) International publication number: WO 2021/215462

(57) **Abstract**

It is an object of the present invention to provide a peptide vaccine complexed so that the peptide vaccine can be delivered specifically to the surface of specific immune cells. It is another object of the present invention to provide a method for delivering a peptide vaccine specifically to the surface of specific immune cells. As a result of diligent studies, the inventors of the present invention have shown that the aforementioned objects can be achieved by providing a peptide vaccine combined with an IgG binding peptide capable of binding to an IgG that is an agonist against molecules on the surface of specific immune cells (e.g., dendritic cells).

## Description

### Technical Field

The present invention relates to a new type of cancer peptide vaccine for the treatment or prevention of cancer. More specifically, the present invention relates to a cancer peptide vaccine that can be specifically delivered to immune cells with which the cancer peptide vaccine is intended to come into contact.

### Background Art

A cancer peptide vaccine has been studied as a new treatment method for cancer. Cancer peptide vaccine therapy is a cancer treatment method that aims to prevent or treat cancer by activating and proliferating a specific immune response against a cancer antigen expressed in cancer cells in the patient's body, in which an antigen consisting of a peptide that is a part of the cancer antigen protein is administered as a vaccine.

Among such research, research on many cancer antigens, and development of vaccines targeting them have been conducted since the 1990s. Some cancer peptide vaccines have been reported to have an effect of inducing immunity specific to cancer at the laboratory level (Non Patent Literature 1: Fifis T. et al., Vaccine. 2004 Nov 25; 23 (2): 258-66). Further, some cancer vaccines have been reported to have exploratory efficacy at an early stage in clinical trials such as induction of immunity and prolongation of survival period (Non Patent Literature 2: Cancer Sci. 2018 Sep; 109 (9): 2660-2669 and Non Patent Literature 3: Cancer Sci. 2017 Dec; 108 (12): 2430-2437).

However, none of these cancer vaccines have been proved to have sufficient efficacy (therapeutic effect) in a confirmatory clinical trial, and none have been approved yet. One of the reasons why the efficacy has not been obtained is considered to be that the delivery of the vaccines to immune cells or activation of immune cells are insufficient.

### Citation List

### Patent Literatures

Patent Literature 1: WO2016/186206
Patent Literature 2: WO2018/230257

### Non Patent Literatures

Non Patent Literature 1: Fifis T. et al., Vaccine. 2004 Nov 25; 23 (2): 258-66
Non Patent Literature 2: Cancer Sci. 2018 Sep; 109 (9): 2660-2669 Non Patent Literature 3: Cancer Sci. 2017 Dec; 108 (12): 2430-2437

### Summary of Invention

### Technical Problem

It is an object of the present invention to provide a peptide vaccine that are complexed so that the peptide vaccine can be delivered specifically to the surface of specific immune cells. It is another object of the present invention to provide a method for delivering a peptide vaccine specifically to the surface of specific immune cells.

### Solution to Problem

As a result of diligent studies, the inventors of the present invention have shown that the aforementioned objects can be achieved by providing a peptide vaccine combined with an IgG binding peptide that is capable of binding to an IgG that is an antibody against molecules on the surface of specific immune cells (e.g., dendritic cells).

More specifically, the present application provides the following aspects in order to achieve the aforementioned objects:
[1]: A peptide vaccine combined with an IgG binding peptide;
[2]: The peptide vaccine according to [1], wherein the peptide vaccine is selected from the group consisting of a cancer vaccine and an infectious disease vaccine;
[3]: The peptide vaccine according to [2], wherein the peptide vaccine is a cancer vaccine selected from the group consisting of a cancer-associated antigen, a cancer neoantigen, and a cancer personalized neoantigen;
[4]: The peptide vaccine according to any one of [1] to [3], wherein the IgG binding peptide is selected from the group consisting of:
   Xaa1 Xaa2 Xaa3 Cys Xaa4 Xaa5 His Xaa6 Gly Xaa7 Leu Val Trp Cys Xaa8 Xaa9 Xaa10 (SEQ ID NO: 1), wherein
   each of Xaa1, Xaa2, Xaa9, and Xaa10 is any amino acid other than Cys or is absent, or Xaa1 and Xaa2 are combined to form Arg Arg Gly Pro,
   each of Xaa3, Xaa4, Xaa5, and Xaa8 is any amino acid other than Cys,
   Xaa6 is Lys, Cys, Asp, Glu, Arg, Leu, 2-amino suberic acid, or diaminopropionic acid, and
   Xaa7 is Glu, Asn, or Gln; and
   Xaa11 Xaa12 Asn Met Gln Cys Gln Arg Arg Phe Tyr Glu Ala Leu His Asp Pro Asn Leu Asn Glu Glu Gln Arg Asn Ala Xaa13 Ile Xaa14 Ser Ile Arg Asp Asp Cys (SEQ ID NO: 2), wherein
   Xaa11 is selected from the group consisting of Gly, Ala, Val, Leu, Ile, Met, Pro, Phe, Trp, Lys, ornithine, Cys, Asp, Glu, beta-Ala, 2-amino suberic acid, diaminopropionic acid, and NH2-(PEG)n-CO (n = 1 to 50) or is absent,
   Xaa12 is selected from the group consisting of Lys, ornithine, Cys, Asp, Glu, Phe, 2-amino suberic acid, and diaminopropionic acid, and
   Xaa13 and Xaa14 are each independently selected from the group consisting of Arg, His, Asp, Glu, Ser, Thr, Asn, Gln, Tyr, and Cys;
[5]: The peptide vaccine according to any one of [1] to [4], wherein Xaa4 is selected from the group consisting of Ala, Ser, and Thr, and Xaa5 is Tyr or Trp;
[6]: The peptide vaccine according to [4], wherein the IgG binding peptide is selected from the group consisting of:
   Asp Cys Ala Tyr His Xaa6 Gly Glu Leu Val Trp Cys Thr (SEQ ID NO: 3) 
Gly Pro Asp Cys Ala Tyr His Xaa6 Gly Glu Leu Val Trp Cys Thr
Phe His (SEQ ID NO: 4)
 Arg Cys Ala Tyr His Xaa6 Gly Glu Leu Val Trp Cys Ser (SEQ ID NO: 5) 
Gly Pro Arg Cys Ala Tyr His Xaa6 Gly Glu Leu Val Trp Cys Ser
Phe His (SEQ ID NO: 6)
 
Ser Pro Asp Cys Ala Tyr His Xaa6 Gly Glu Leu Val Trp Cys Thr
Phe His (SEQ ID NO: 7)
 
Gly Asp Asp Cys Ala Tyr His Xaa6 Gly Glu Leu Val Trp Cys Thr
Phe His (SEQ ID NO: 8)
 
Gly Pro Ser Cys Ala Tyr His Xaa6 Gly Glu Leu Val Trp Cys Thr
Phe His (SEQ ID NO: 9)
 
Gly Pro Asp Cys Ala Tyr His Xaa6 Gly Glu Leu Val Trp Cys Ser
Phe His (SEQ ID NO: 10)
 
Gly Pro Asp Cys Ala Tyr His Xaa6 Gly Glu Leu Val Trp Cys Thr
His His (SEQ ID NO: 11)
 
Gly Pro Asp Cys Ala Tyr His Xaa6 Gly Glu Leu Val Trp Cys Thr
Phe Tyr (SEQ ID NO: 12)
 
Ser Pro Asp Cys Ala Tyr His Xaa6 Gly Glu Leu Val Trp Cys Thr
Phe Tyr (SEQ ID NO: 13)
 
Ser Asp Asp Cys Ala Tyr His Xaa6 Gly Glu Leu Val Trp Cys Thr
Phe Tyr (SEQ ID NO: 14)
 
Arg Gly Asn Cys Ala Tyr His Xaa6 Gly Gln Leu Val Trp Cys Thr
Tyr His (SEQ ID NO: 15)
 
Gly Xaa2 Asp Cys Ala Tyr His Xaa6 Gly Glu Leu Val Trp Cys Thr
Xaa9 His (SEQ ID NO: 16)
 
Arg Arg Gly Pro Asp Cys Ala Tyr His Xaa6 Gly Glu Leu Val Trp
Cys Thr Phe His (SEQ ID NO: 17)
 
Phe Asn Met Gln Cys Gln Arg Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 18),
 
Gly Phe Asn Met Gln Cys Gln Arg Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 19),
 
Lys Asn Met Gln Cys Gln Arg Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 20),
 
Gly Phe Asn Met Gln Cys Gln Lys Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 21),
 
Lys Asn Met Gln Cys Gln Lys Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 22);
 
Phe Asn Met Gln Gln Gln Arg Arg Phe Tyr Glu Ala Leu His Asp
Pro Asn Leu Asn Glu Glu Gln Arg Asn Ala Arg Ile Arg Ser Ile Arg
Asp Asp (SEQ ID NO: 23); and
 
Gly Lys Asn Met Gln Cys Gln Arg Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 24);
[7]: The peptide vaccine according to [6], wherein the IgG binding peptide is 
Gly Pro Asp Cys Ala Tyr His Arg Gly Glu Leu Val Trp Cys Thr
Phe His(SEQ ID NO: 25)
 
Gly Pro Asp Cys Ala Tyr His Lys Gly Glu Leu Val Trp Cys Thr
Phe His(SEQ ID NO: 26)
 
Gly Pro Asp Cys Ala Trp His Arg Gly Glu Leu Val Trp Cys Thr
Phe His(SEQ ID NO: 39)
 
Gly Pro Asp Cys Ala Trp His Leu Gly Glu Leu Val Trp Cys Thr
Phe His(SEQ ID NO: 40);
[8]: The peptide vaccine according to any one of [1] to [7], wherein an IgG is bound to the IgG binding peptide;
[9]: The peptide vaccine according to [8], wherein the IgG is an antibody against a target substance characteristic to immune cells which delivers the peptide vaccine;
[10]: The peptide vaccine according to [9], wherein the immune cells are selected from the group consisting of antigen-presenting cells, dendritic cells, and B cells;
[11]: The peptide vaccine according to any one of [8] to [10], wherein the IgG is selected from the group consisting of an anti-CD40 antibody, an anti-PD-L1 antibody, an anti-DEC205 antibody, an anti-DCIR antibody, an anti-Mannose receptor antibody, an anti-DC-SIGN antibody, an anti-CD11c antibody, and an anti-Dectin-1 antibody;
[12]: A method of delivering a peptide vaccine to cells having molecules targeted by the IgG on the cell surface by using an IgG, an IgG binding peptide, and a peptide vaccine combined with the IgG binding peptide;
[13]: The method according to [12], wherein the peptide vaccine is selected from the group consisting of a cancer vaccine and an infectious disease vaccine;
[14]: The method according to [13], wherein the peptide vaccine is a cancer vaccine selected from the group consisting of a cancer-associated antigen, a cancer neoantigen, and a cancer personalized neoantigen;
[15]: The method according to any one of [12] to [14], wherein the IgG binding peptide is selected from the group consisting of: 
Xaa1 Xaa2 Xaa3 Cys Xaa4 Xaa5 His Xaa6 Gly Xaa7 Leu Val Trp
Cys Xaa8 Xaa9 Xaa10 (SEQ ID NO: 1), wherein
 wherein
   each of Xaa1, Xaa2, Xaa9, and Xaa10 is any amino acid other than Cys or is absent, or Xaa1 and Xaa2 are combined to form Arg Arg Gly Pro,
   each of Xaa3, Xaa4, Xaa5, and Xaa8 is any amino acid other than Cys,
   Xaa6 is Lys, Cys, Asp, Glu, Arg, Leu, 2-amino suberic acid, or diaminopropionic acid, and
   Xaa7 is Glu, Asn, or Gln; and
   Xaa11 Xaa12 Asn Met Gln Cys Gln Arg Arg Phe Tyr Glu Ala Leu His Asp Pro Asn Leu Asn Glu Glu Gln Arg Asn Ala Xaa13 Ile Xaa14 Ser Ile Arg Asp Asp Cys (SEQ ID NO: 2), wherein
   Xaa11 is selected from the group consisting of Gly, Ala, Val, Leu, Ile, Met, Pro, Phe, Trp, Lys, ornithine, Cys, Asp, Glu, beta-Ala, 2-amino suberic acid, diaminopropionic acid, and NH2-(PEG)n-CO (n = 1 to 50) or is absent,
   Xaa12 is selected from the group consisting of Lys, ornithine, Cys, Asp, Glu, Phe, 2-amino suberic acid, and diaminopropionic acid, and
   Xaa13 and Xaa14 are each independently selected from the group consisting of Arg, His, Asp, Glu, Ser, Thr, Asn, Gln, Tyr, and Cys;
[16]: The method according to any one of [12] to [15], wherein Xaa4 is selected from the group consisting of Ala, Ser, and Thr, and Xaa5 is Tyr or Trp.
[17]: The method according to [15], wherein the IgG binding peptide is selected from the group consisting of:
   Asp Cys Ala Tyr His Xaa6 Gly Glu Leu Val Trp Cys Thr (SEQ ID NO: 3) 
Gly Pro Asp Cys Ala Tyr His Xaa6 Gly Glu Leu Val Trp Cys Thr
Phe His (SEQ ID NO: 4)
 Arg Cys Ala Tyr His Xaa6 Gly Glu Leu Val Trp Cys Ser (SEQ ID NO: 5) 
Gly Pro Arg Cys Ala Tyr His Xaa6 Gly Glu Leu Val Trp Cys Ser
Phe His (SEQ ID NO: 6)
 
Ser Pro Asp Cys Ala Tyr His Xaa6 Gly Glu Leu Val Trp Cys Thr
Phe His (SEQ ID NO: 7)
 
Gly Asp Asp Cys Ala Tyr His Xaa6 Gly Glu Leu Val Trp Cys Thr
Phe His (SEQ ID NO: 8)
 
Gly Pro Ser Cys Ala Tyr His Xaa6 Gly Glu Leu Val Trp Cys Thr
Phe His (SEQ ID NO: 9)
 
Gly Pro Asp Cys Ala Tyr His Xaa6 Gly Glu Leu Val Trp Cys Ser
Phe His (SEQ ID NO: 10)
 
Gly Pro Asp Cys Ala Tyr His Xaa6 Gly Glu Leu Val Trp Cys Thr
His His (SEQ ID NO: 11)
 
Gly Pro Asp Cys Ala Tyr His Xaa6 Gly Glu Leu Val Trp Cys Thr
Phe Tyr (SEQ ID NO: 12)
 
Ser Pro Asp Cys Ala Tyr His Xaa6 Gly Glu Leu Val Trp Cys Thr
Phe Tyr (SEQ ID NO: 13)
 
Ser Asp Asp Cys Ala Tyr His Xaa6 Gly Glu Leu Val Trp Cys Thr
Phe Tyr (SEQ ID NO: 14)
 
Arg Gly Asn Cys Ala Tyr His Xaa6 Gly Gln Leu Val Trp Cys Thr
Tyr His (SEQ ID NO: 15)
 
Gly Xaa2 Asp Cys Ala Tyr His Xaa6 Gly Glu Leu Val Trp Cys Thr
Xaa9 His (SEQ ID NO: 16)
 
Arg Arg Gly Pro Asp Cys Ala Tyr His Xaa6 Gly Glu Leu Val Trp
Cys Thr Phe His (SEQ ID NO: 17)
 
Phe Asn Met Gln Cys Gln Arg Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 18),
 
Gly Phe Asn Met Gln Cys Gln Arg Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 19),
 
Lys Asn Met Gln Cys Gln Arg Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 20),
 
Gly Phe Asn Met Gln Cys Gln Lys Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 21),
 
Lys Asn Met Gln Cys Gln Lys Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 22);
 
Phe Asn Met Gln Gln Gln Arg Arg Phe Tyr Glu Ala Leu His Asp
Pro Asn Leu Asn Glu Glu Gln Arg Asn Ala Arg Ile Arg Ser Ile Arg
Asp Asp (SEQ ID NO: 23); and
 
Gly Lys Asn Met Gln Cys Gln Arg Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 24);
[18]: The method according to [17], wherein the IgG binding peptide is: 
Gly Pro Asp Cys Ala Tyr His Arg Gly Glu Leu Val Trp Cys Thr
Phe His (SEQ ID NO: 25);
 
Gly Pro Asp Cys Ala Tyr His Lys Gly Glu Leu Val Trp Cys Thr
Phe His (SEQ ID NO: 26);
 
Gly Pro Asp Cys Ala Trp His Arg Gly Glu Leu Val Trp Cys Thr
Phe His (SEQ ID NO: 39); or
 or 
Gly Pro Asp Cys Ala Trp His Leu Gly Glu Leu Val Trp Cys Thr
Phe His (SEQ ID NO: 40);
[19]: The method according to any one of [12] to [18], wherein the IgG is an antibody against a target substance characteristic to immune cells which delivers the peptide vaccine;
[20]: The method according to [19], wherein the immune cells are selected from the group consisting of antigen-presenting cells, dendritic cells, and B cells; and
[21]: The method according to [19] or [20], wherein the IgG is selected from the group consisting of an anti-CD40 antibody, an anti-PD-L1 antibody, an anti-DEC205 antibody, an anti-DCIR antibody, an anti-Mannose receptor antibody, an anti-DC-SIGN antibody, an anti-CD11c antibody, and an anti-Dectin-1 antibody.

### Advantageous Effects of Invention

The peptide vaccine combined with an IgG binding peptide disclosed in the present invention enables the peptide vaccine to be efficiently delivered to the surface of specific immune cells (e.g., dendritic cells) and enhances the activation to improve the effects of the peptide vaccine.

### Brief Description of Drawings

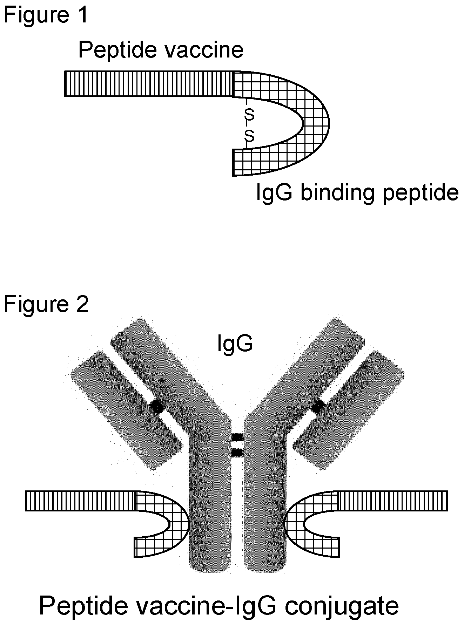

[Figure 1] Figure 1 is a schematic diagram showing a peptide vaccine combined with an IgG binding peptide.
[Figure 2] Figure 2 is a schematic diagram showing a conjugate (peptide vaccine-IgG conjugate) in which the peptide vaccine combined with an IgG binding peptide is bound to IgG via an IgG binding peptide moiety.

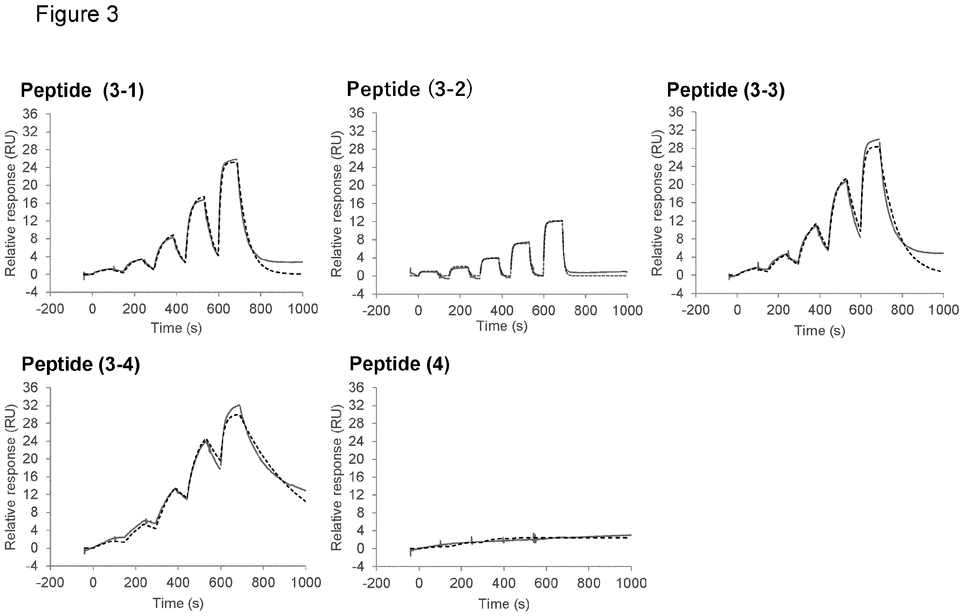

[Figure 3] Figure 3 is a figure which shows the results of affinity analysis of peptide for IgG, in which the affinities of H-2Kb-restricted OVA-derived peptides having various IgG binding peptides prepared in Example 3 for IgG were examined in vivo.

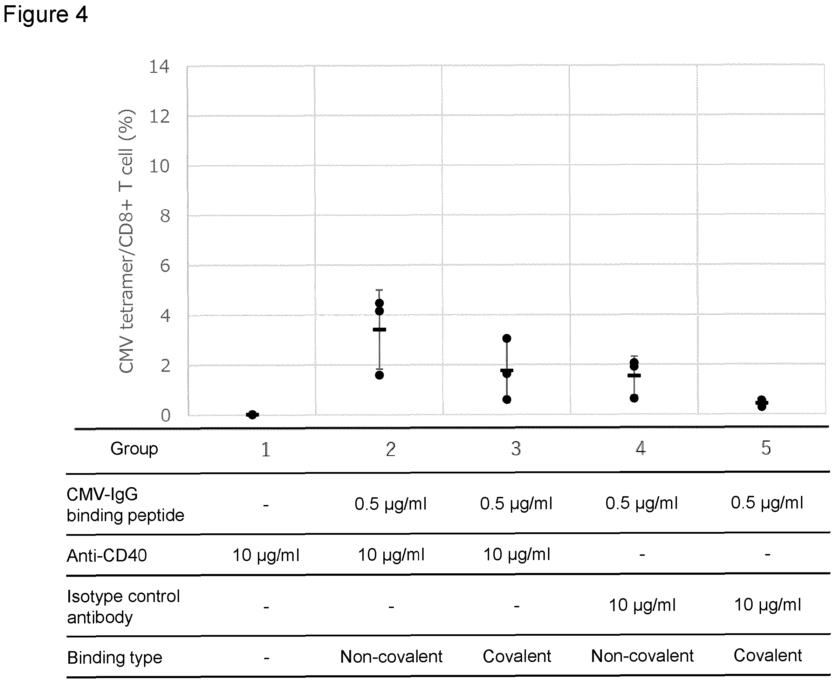

[Figure 4] Figure 4 is a figure which shows the results of the in-vitro immunity induction test (whole PBMC method), which examined in vitro whether or not the antibody-peptide vaccine conjugates (covalently or non-covalently bonded) using peptide vaccines prepared in Example 3 had an ability to induce immunity.

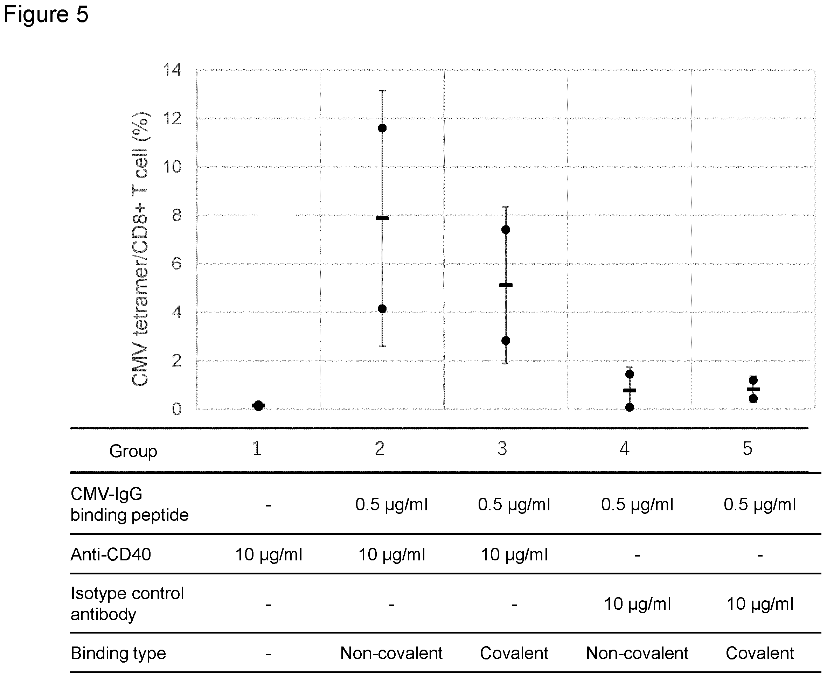

[Figure 5] Figure 5 is a figure which shows the results of the in-vitro immunity induction test (monocyte-derived dendritic cell (MDDC) method), which examined in vitro whether or not the antibody-peptide vaccine conjugates (covalently or non-covalently bonded) using peptide vaccines prepared in Example 3 had an ability to induce immunity.

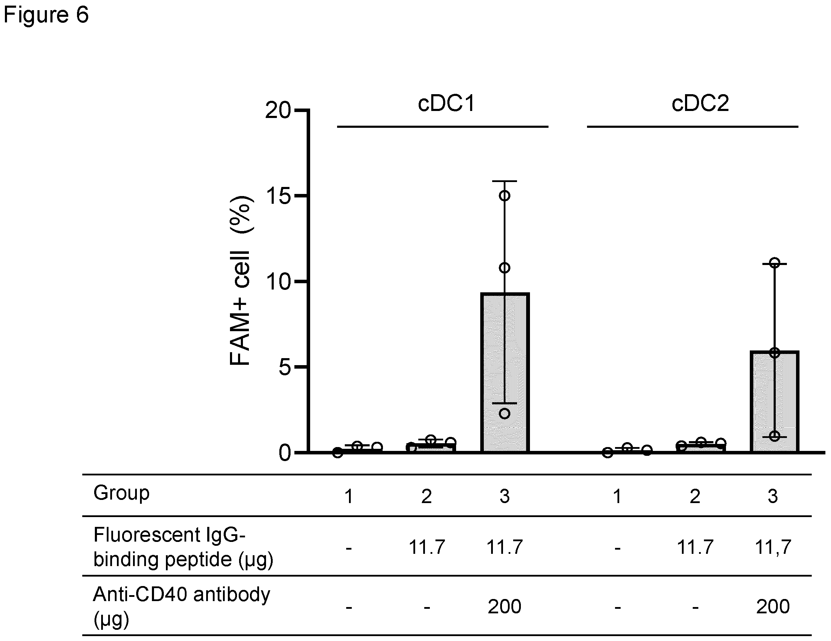

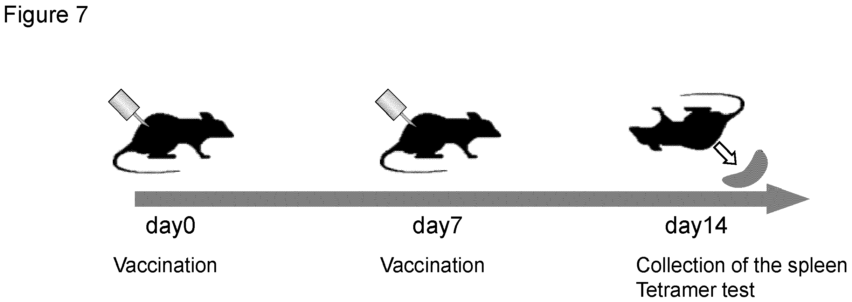

[Figure 6] Figure 6 is a figure which shows the results of the evaluation of peptide uptake into dendritic cells in wild-type mice, which examined in vivo whether or not the antibody-peptide conjugates were incorporated with dendritic cells when the antibody-peptide conjugates produced using the peptides prepared in Example 3 are administered subcutaneously. [Figure 7] Figure 7 is a figure which illustrates a schedule of the immunity induction test in wild-type mice.

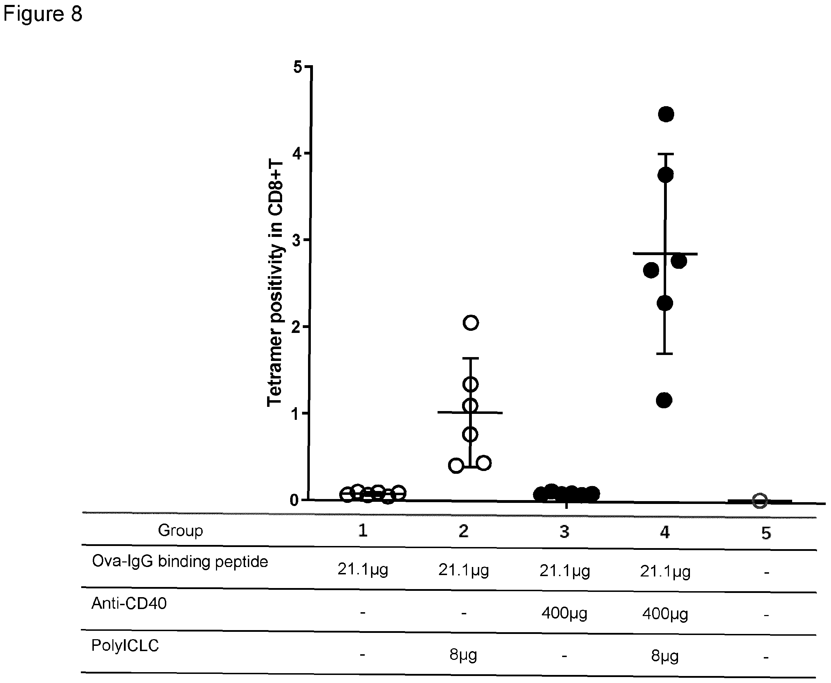

[Figure 8] Figure 8 is a figure which shows the results of the immunity induction test in wild-type mice, which examined in vivo whether or not the antibody-peptide vaccine conjugates (non-covalently bonded) produced using peptide vaccines prepared in Example 3 had an ability to induce immunity.

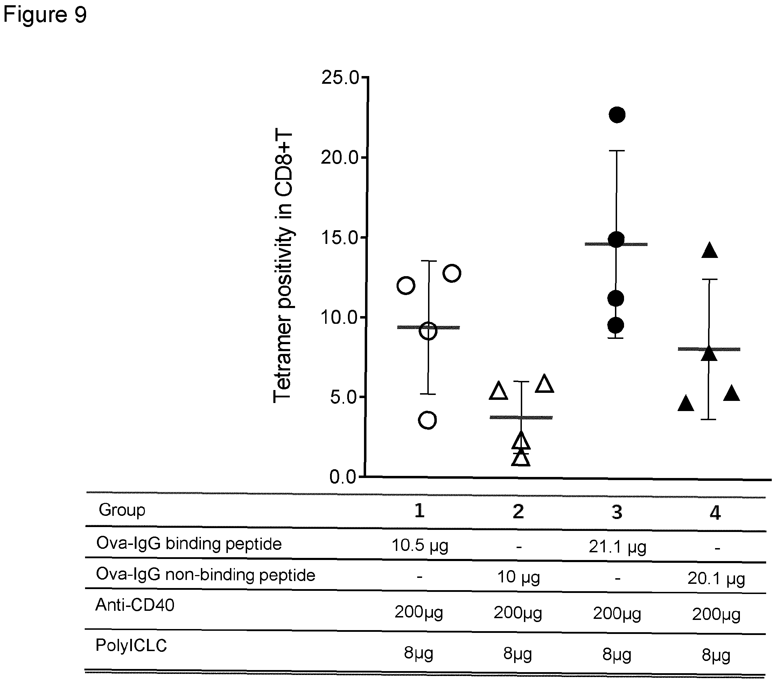

[Figure 9] Figure 9 is a figure which shows the results of the immunity induction test (comparison test for antibody-vaccine mixing ratio) in wild-type mice, which examined in vivo whether or not the antibody-peptide vaccine conjugates (non-covalently bonded) produced using peptide vaccines prepared in Example 3 had an ability to induce immunity.

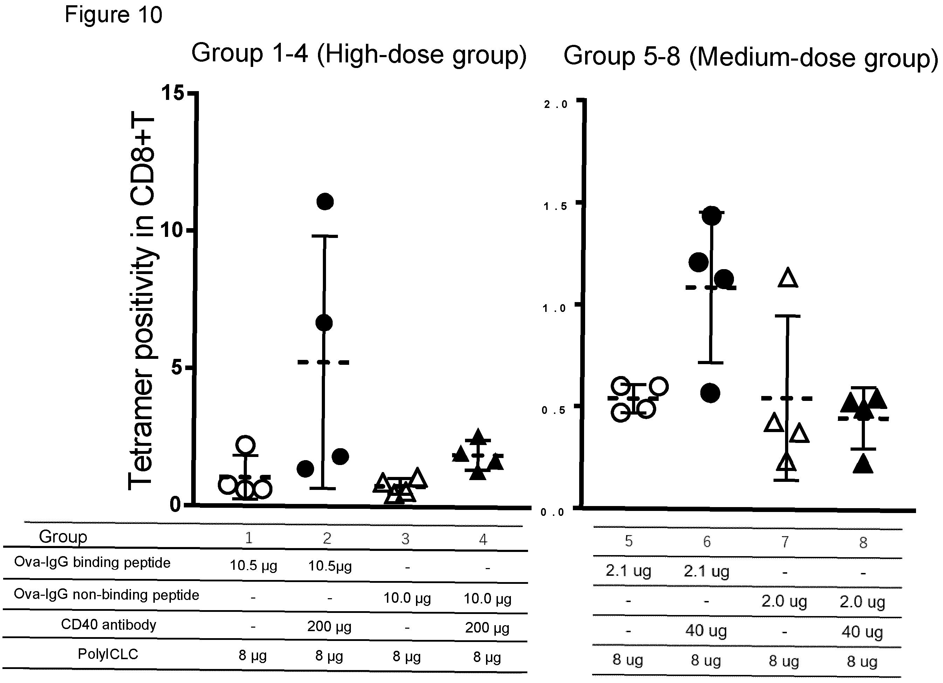

[Figure 10] Figure 10 is a figure which shows the results of the immunity induction test (dose-dependency test) in wild-type mice, which examined in vivo whether or not the antibody-peptide vaccine conjugates (non-covalently bonded) produced using peptide vaccines prepared in Example 3 had an ability to induce immunity.

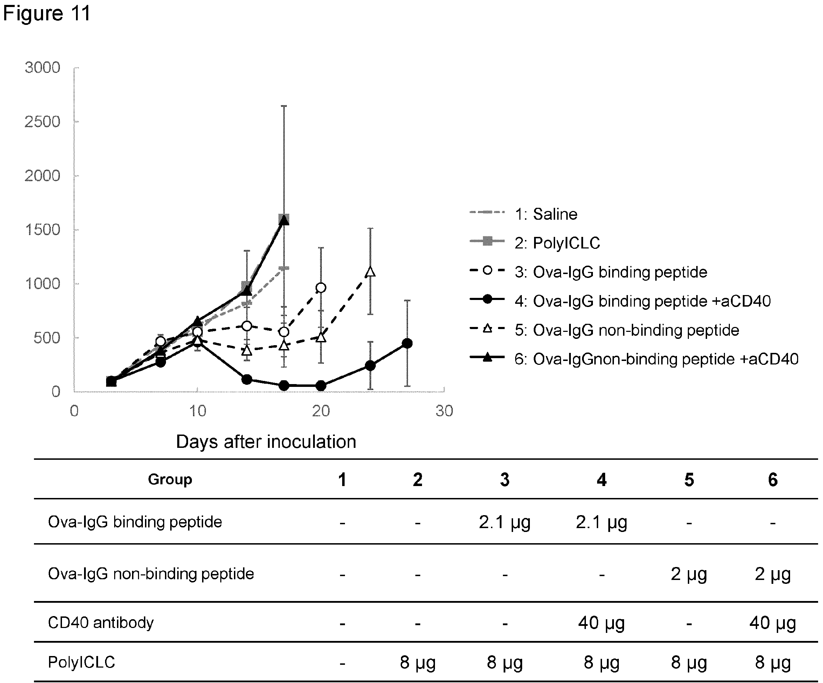

[Figure 11] Figure 11 is a figure which shows the results of the cancer-bearing mouse test, which examined in vivo whether or not the antibody-peptide vaccine conjugates (non-covalently bonded) produced using peptide vaccines prepared in Example 3 had an anti-tumor effect. In this figure, the antibody:antigen peptide-IgG binding peptide group exhibited the highest tumor growth inhibitory effect also in vivo.

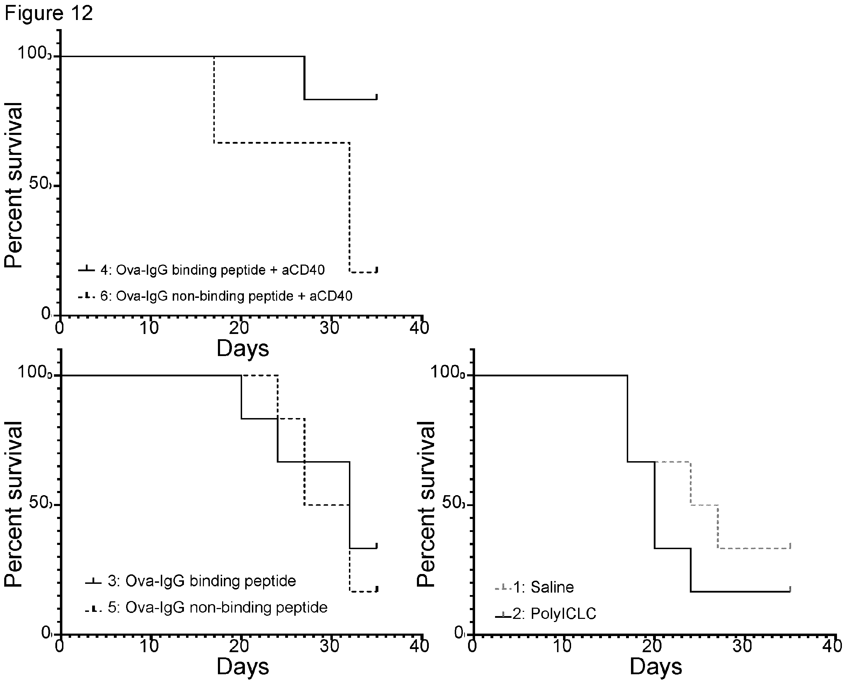

[Figure 12] Figure 12 is a figure which shows the results of the cancer-bearing mouse test, which examined in vivo whether or not the antibody-peptide vaccine conjugates (non-covalently bonded) produced using peptide vaccines prepared in Example 3 had an anti-tumor effect. In this figure, the antibody:antigen peptide-IgG binding peptide group exhibited a remarkable prolonging effect on the survival period.

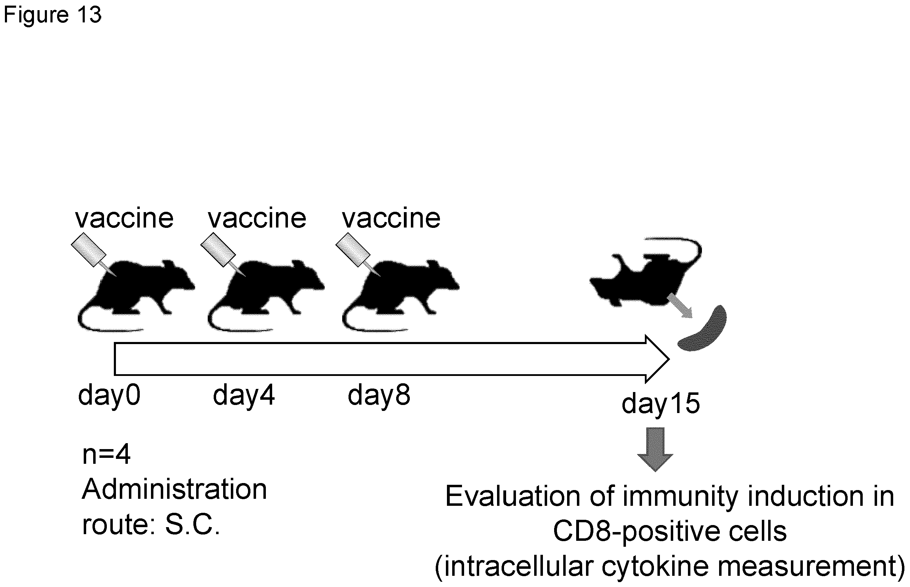

[Figure 13] Figure 13 is a figure which illustrates a schedule of the immunity induction test in wild-type mice.

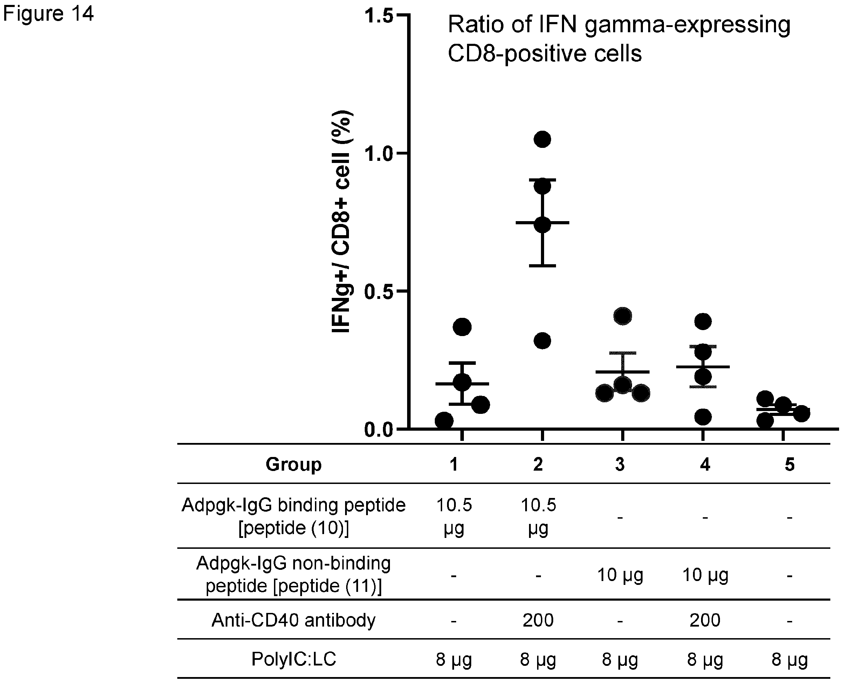

[Figure 14] Figure 14 is a figure which shows the results of the immunity induction test (neoantigen model) in wild-type mice, which examined, in a test system with higher clinical extrapolation, whether or not the antibody-peptide vaccine conjugates (non-covalently bonded) produced using peptide vaccines prepared in Example 3 had an ability to induce immunity. In this figure, the antibody: antigen-IgG binding peptide group exhibited the highest immunity induction effect.

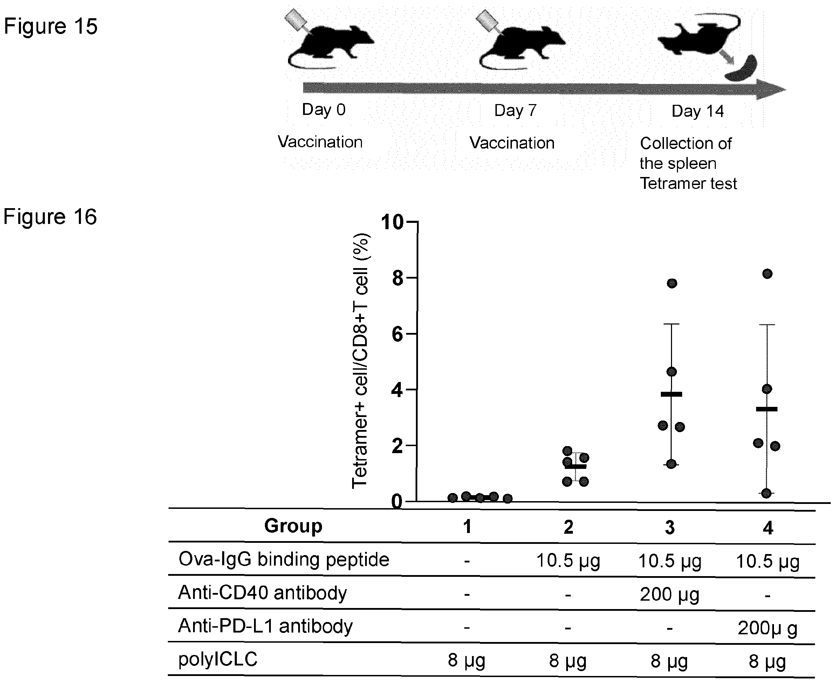

[Figure 15] Figure 15 is a figure which illustrates a schedule of the immunity induction test in wild-type mice.
[Figure 16] Figure 16 is a figure which shows the results of the immunity induction test in wild-type mice, which examined in vivo whether or not the antibody-peptide vaccine conjugates (non-covalently bonded) produced using peptide vaccines prepared in Example 3 to be combined with the CD40 antibody or the PD-L1 antibody had an ability to induce immunity.

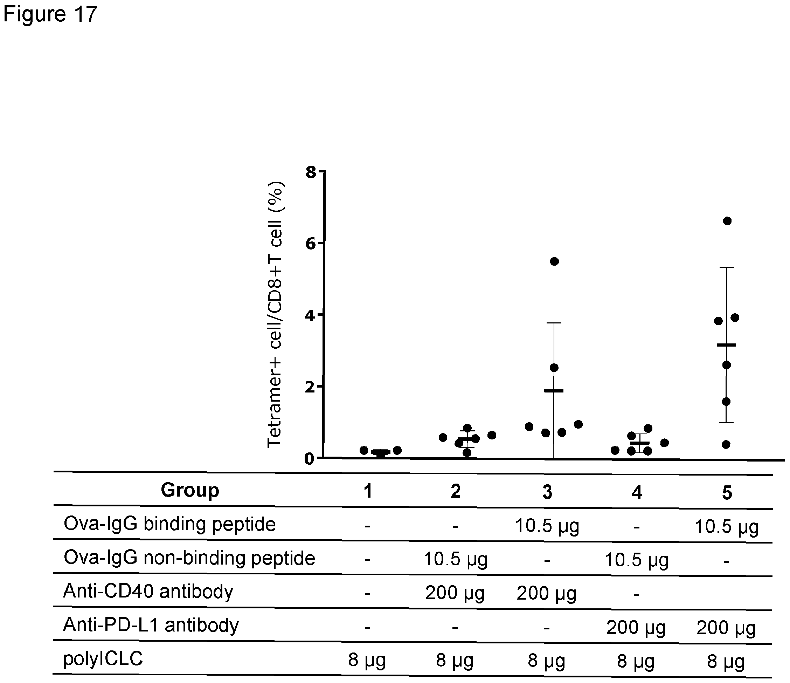

[Figure 17] Figure 17 is a figure which shows the results of the immunity induction test in wild-type mice, which examined in vivo whether or not the antibody-peptide vaccine conjugates (non-covalently bonded) produced using peptide vaccines prepared in Example 3 to be combined with the CD40 antibody or the PD-L1 antibody had an ability to induce immunity.

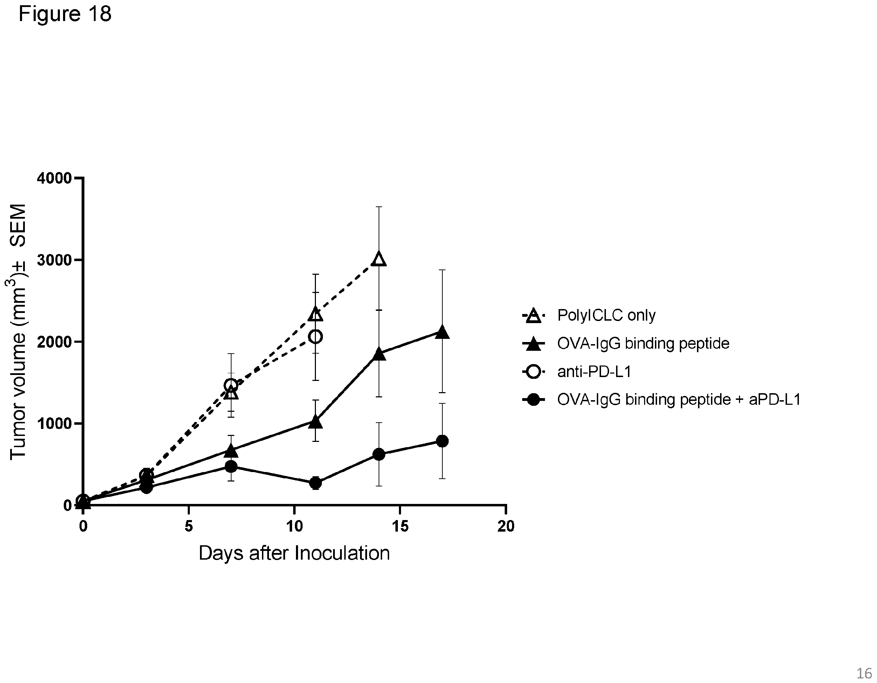

[Figure 18] Figure 18 is a figure which shows the results of the cancer-bearing mouse test, which examined in vivo whether or not the antibody-peptide vaccine conjugates (non-covalently bonded) produced using peptide vaccines prepared in Example 3 to be combined with an anti-PD-L1 antibody had an anti-tumor effect. In this figure, the antibody:antigen peptide-IgG binding peptide group exhibited the highest tumor growth inhibitory effect in vivo.

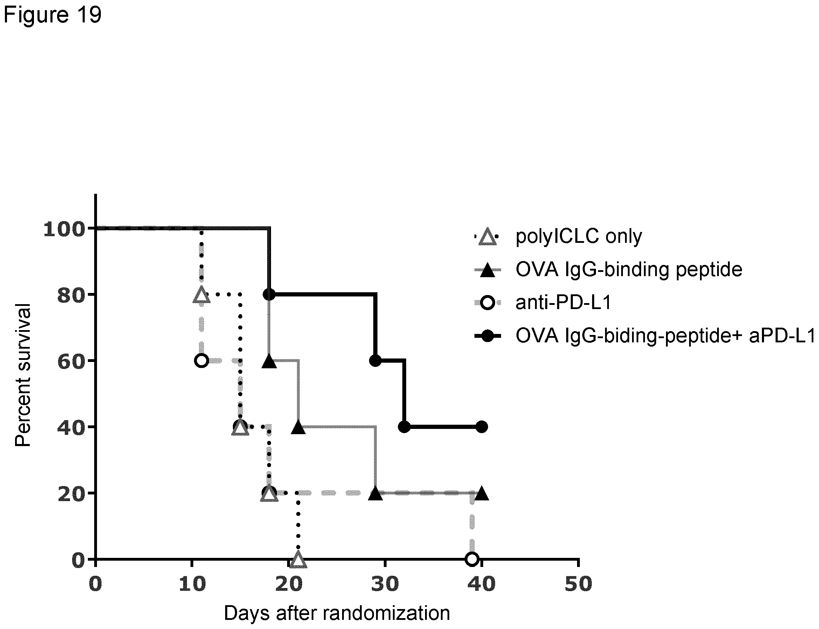

[Figure 19] Figure 19 is a figure which shows the results of the cancer-bearing mouse test, which examined in vivo whether or not the antibody-peptide vaccine conjugates (non-covalently bonded) produced using peptide vaccines prepared in Example 3 had an anti-tumor effect. In this figure, the antibody:antigen peptide-IgG binding peptide group exhibited a remarkable prolonging effect on the survival period.

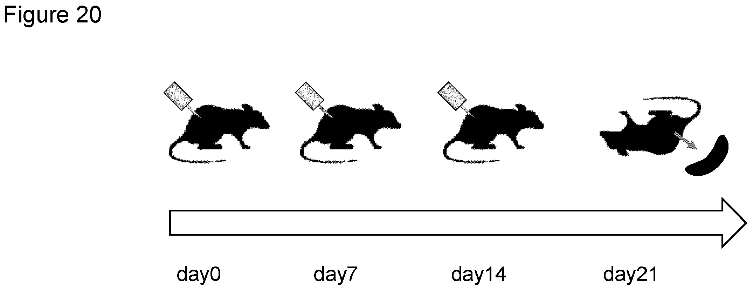

[Figure 20] Figure 20 is a figure which illustrates a schedule of the immunity induction test in wild-type mice.

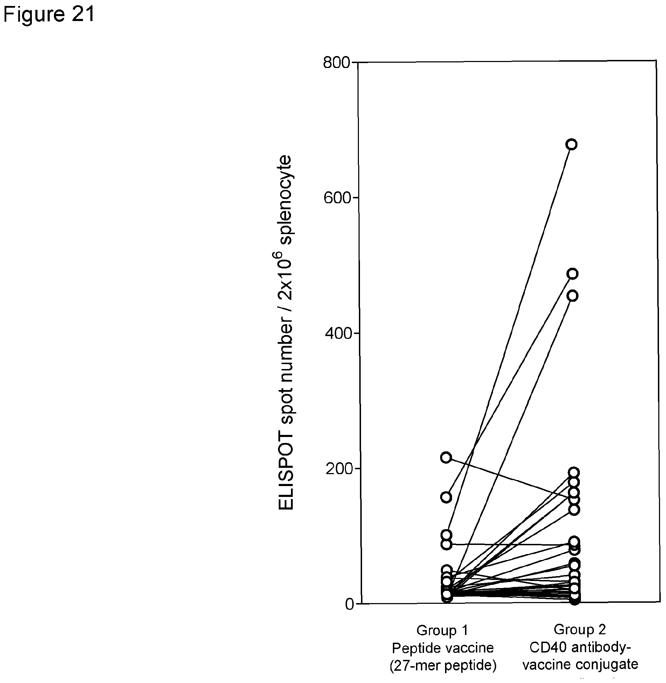

[Figure 21] Figure 21 is a figure which shows the results of the immunity induction test in wild-type mice, which examined in vivo whether or not the antibody-peptide vaccine conjugates (non-covalently bonded) containing the sequence of the non-synonymous somatic mutations of a mouse tumor cell line had an anti-tumor effect.

### Description of Embodiments

The terms to be used in the present invention will be defined as follows:
(a) Cancer vaccine: A therapeutic drug in which all or part of a protein whose expression is enhanced in cancer cells or expressed only in cancer cells (cancer antigen) is administered as a pharmaceutical product to induce the immunity against the cancer antigen and eliminate the cancer;
(b) Dendritic cells: Immune cells which initially recognize foreign substance such as cancer antigens in the induction of the immune system against such foreign substance;
(c) T cells: Immune cells to which a function of specifically killing cancer cells is imparted based on the information on the cancer antigen recognized by dendritic cells;
(d) CD40: One of the proteins expressed on the surface of antigen-presenting cells such as dendritic cells. CD40 can enhance the function of the antigen-presenting cells by binding to its agonist molecules such as ligands through thereon;
(e) Antibody vaccine: A conjugate of vaccine and antibody.

### <Peptide vaccine>

According to one embodiment, the present invention discloses an invention relating to a peptide vaccine combined with an IgG binding peptide (Figure 1). In this embodiment, peptide vaccines can be made of an antigen of any origin as long as they are vaccines using antigenic peptides which can be used as vaccines against pathogens, vaccines against cancer (cancer vaccine), or the like. In the case where vaccines against pathogens are used as peptide vaccines, antigenic peptides of pathogens such as HIV vaccine, HBV vaccine, and RSV vaccine can be used. Further, in the case where cancer vaccines are used as peptide vaccines, antigenic peptides specific to cancer such as brain tumor, myeloma, stomach cancer, colorectal cancer, uterine cancer, head and neck cancer, breast cancer, lung cancer, pancreatic cancer, mesothelioma, ovarian cancer, prostate cancer, melanoma and so on can be used. In general, antigenic peptides derived from cancer antigens that are considered suitable for immunotherapy can be used as antigens for cancer vaccines. It can be selected, for example, from:
- Peptides expressed in specific tumor cells but only in a testis in normal tissues (such as NY-ESO-1, MAGEA1-4, PRAME, SSX2, and CT83),
- Peptides expressed in tumor cells at a much higher level than in normal cells (such as WT1, HER2, CEA, MUC1, cyclin B1, EGFR, mesothelin, and telomerase),
- Peptides expressed in tumor cells and only in a limited lineage of cells in normal tissues (such as MART1, PSA, PSMA, CD19, and GP100),
- Peptides expressed also in normal tissues but specially modified in tumor cells (such as MUC1T and LSP1),
- New proteins that occur specifically in diseases due to abnormalities such as gene translocations and point mutations commonly observed in the diseases (such as BCR-ABL1, HRAS, and KRAS), and
- A group of peptides derived from mutant proteins (neoantigens) that occur only in tumor cells as a result of gene mutations derived from genomic instability that are often observed in tumor cells.

It is known that peptide vaccines, when administered alone, often fail to sufficiently induce immunity. The reason for this is thought to be the inability to deliver the peptide vaccines to the target immune cells. The peptide vaccine in the present invention provides a peptide vaccine combined with an IgG binding peptide in order to use IgG for delivering the peptide vaccine to the target immune cells for the purpose of compensating for such a commonly seen disadvantage of peptide vaccines.

As the IgG binding peptide to be used in the present invention, a known peptide can be used which is capable of binding IgG to the peptide of the peptide vaccine. Examples thereof can include, but not limited to, the IgG binding peptide described in WO2016/186206 (Patent Literature 1) (type 1) and the IgG binding peptide described in WO2018/230257 (Patent Literature 2) (type 2). The present invention can provide a peptide vaccine combined with such an IgG binding peptide. The IgG binding peptide that can be used in one embodiment of the present invention is selected from the group consisting of:
<Type 1> 
Xaa1 Xaa2 Xaa3 Cys Xaa4 Xaa5 His Xaa6 Gly Xaa7 Leu Val Trp
Cys Xaa8 Xaa9 Xaa10 (SEQ ID NO: 1), wherein
 wherein
   each of Xaa1, Xaa2, Xaa9, and Xaa10 is any amino acid other than Cys or is absent, or Xaa1 and Xaa2 are combined to form Arg Arg Gly Pro,
   each of Xaa3, Xaa4, Xaa5, and Xaa8 is any amino acid other than Cys, more preferably, Xaa4 may be selected from the group consisting of Ala, Ser, and Thr, and Xaa5 may be Tyr or Trp,
   Xaa6 is Lys, Cys, Asp, Glu, Arg, Leu, 2-amino suberic acid, or diaminopropionic acid, and
   Xaa7 is Glu, Asn, or Gin; and
<Type 2> 
Xaa11 Xaa12 Asn Met Gln Cys Gln Arg Arg Phe Tyr Glu Ala Leu
His Asp Pro Asn Leu Asn Glu Glu Gln Arg Asn Ala Xaa13 Ile Xaa14
Ser Ile Arg Asp Asp Cys (SEQ ID NO: 2), wherein
 wherein
   Xaa11 is selected from the group consisting of Gly, Ala, Val, Leu, Ile, Met, Pro, Phe, Trp, Lys, ornithine, Cys, Asp, Glu, beta-Ala, 2-amino suberic acid, diaminopropionic acid, and NH2-(PEG)n-CO (n = 1 to 50) or is absent,
   Xaa12 is selected from the group consisting of Lys, ornithine, Cys, Asp, Glu, Phe, 2-amino suberic acid, and diaminopropionic acid, and
   Xaa13 and Xaa14 are each independently selected from the group consisting of Arg, His, Asp, Glu, Ser, Thr, Asn, Gln, Tyr, and Cys;
   and such an IgG binding peptide can be used in combination with a peptide vaccine.

More specifically, the IgG binding peptide that can be used in one embodiment of the present invention is selected from the group consisting of:
<Type 1>:
   Asp Cys Ala Tyr His Xaa6 Gly Glu Leu Val Trp Cys Thr (SEQ ID NO: 3); 
Gly Pro Asp Cys Ala Tyr His Xaa6 Gly Glu Leu Val Trp Cys Thr
Phe His (SEQ ID NO: 4);
 Arg Cys Ala Tyr His Xaa6 Gly Glu Leu Val Trp Cys Ser (SEQ ID NO: 5) ; 
Gly Pro Arg Cys Ala Tyr His Xaa6 Gly Glu Leu Val Trp Cys Ser
Phe His (SEQ ID NO: 6);
 
Ser Pro Asp Cys Ala Tyr His Xaa6 Gly Glu Leu Val Trp Cys Thr
Phe His (SEQ ID NO: 7);
 
Gly Asp Asp Cys Ala Tyr His Xaa6 Gly Glu Leu Val Trp Cys Thr
Phe His (SEQ ID NO: 8);
 
Gly Pro Ser Cys Ala Tyr His Xaa6 Gly Glu Leu Val Trp Cys Thr
Phe His (SEQ ID NO: 9);
 
Gly Pro Asp Cys Ala Tyr His Xaa6 Gly Glu Leu Val Trp Cys Ser
Phe His (SEQ ID NO: 10);
 
Gly Pro Asp Cys Ala Tyr His Xaa6 Gly Glu Leu Val Trp Cys Thr
His His (SEQ ID NO: 11);
 
Gly Pro Asp Cys Ala Tyr His Xaa6 Gly Glu Leu Val Trp Cys Thr
Phe Tyr (SEQ ID NO: 12);
 
Ser Pro Asp Cys Ala Tyr His Xaa6 Gly Glu Leu Val Trp Cys Thr
Phe Tyr (SEQ ID NO: 13);
 
Ser Asp Asp Cys Ala Tyr His Xaa6 Gly Glu Leu Val Trp Cys Thr
Phe Tyr (SEQ ID NO: 14);
 
Arg Gly Asn Cys Ala Tyr His Xaa6 Gly Gln Leu Val Trp Cys Thr
Tyr His (SEQ ID NO: 15);
 
Gly Xaa2 Asp Cys Ala Tyr His Xaa6 Gly Glu Leu Val Trp Cys Thr
Xaa9 His (SEQ ID NO: 16);
 
Arg Arg Gly Pro Asp Cys Ala Tyr His Xaa6 Gly Glu Leu Val Trp
Cys Thr Phe His (SEQ ID NO: 17); and
<Type 2>:

Phe Asn Met Gln Cys Gln Arg Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 18);

Gly Phe Asn Met Gln Cys Gln Arg Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 19);

Lys Asn Met Gln Cys Gln Arg Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 20);

Gly Phe Asn Met Gln Cys Gln Lys Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 21);

Lys Asn Met Gln Cys Gln Lys Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 22);

Phe Asn Met Gln Gln Gln Arg Arg Phe Tyr Glu Ala Leu His Asp
Pro Asn Leu Asn Glu Glu Gln Arg Asn Ala Arg Ile Arg Ser Ile Arg
Asp Asp (SEQ ID NO: 23);

Gly Lys Asn Met Gln Cys Gln Arg Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 24).

Among these, the IgG binding peptide in which Xaa6 is Arg, Lys, or Leu is particularly useful in the present invention, and specific examples of the amino acid sequence of the useful IgG binding peptide can include

Gly Pro Asp Cys Ala Tyr His Arg Gly Glu Leu Val Trp Cys Thr
Phe His (SEQ ID NO: 25),

Gly Pro Asp Cys Ala Tyr His Lys Gly Glu Leu Val Trp Cys Thr
Phe His (SEQ ID NO: 26),

Gly Pro Asp Cys Ala Trp His Arg Gly Glu Leu Val Trp Cys Thr
Phe His (SEQ ID NO: 39), or

Gly Pro Asp Cys Ala Trp His Leu Gly Glu Leu Val Trp Cys Thr
Phe His (SEQ ID NO: 40).

The IgG binding peptide of the present invention is preferably intended for binding to human IgG (e.g., any of the IgG subclasses of human IgG1, IgG2, IgG3, and IgG4) or humanized IgG, but can be used for IgG in animals other than human, such as rats, mice, and rabbits.

The method for combining the IgG binding peptide with a peptide vaccine may be any method. It can be produced by a method of combining the IgG binding peptide and the peptide of the peptide vaccine to produce one continuous peptide using a peptide synthesis method such as a conventional liquid-phase synthesis method and a solid-phase synthesis method, peptide synthesis by an automatic peptide synthesizer, and the like, or a method of combining the IgG binding peptide and the peptide of the peptide vaccine synthesized separately via a linker. Alternatively, the peptide may be produced by a gene recombination method using a nucleic acid encoding the peptide of the present invention. For example, the target peptide can be produced by incorporating a DNA encoding the amino acid sequence of one contiguous peptide with the peptide of the IgG binding peptide of the present invention and the peptide of the peptide vaccine into an expression vector and introducing it into the host cells, followed by culture.

In consideration of the ease of synthesis or the like, the combination method is preferably synthesis of the IgG binding peptide and the peptide of the peptide vaccine as one contiguous peptide. In the case of employing such a method, a desired sequence of the peptide vaccine combined with an IgG binding peptide can be designed, and the peptide can be synthesized using a peptide synthesizer (e.g., Symphony (R) X, available from Gyros Protein Technologies AB). The contaminants contained when synthesizing the peptide can be removed, for example, by chromatography such as gel filtration chromatography, ion-exchange column chromatography, affinity chromatography, and reverse phase column chromatography, ammonium sulfate fractionation, ultrafiltration, and the immunoadsorption method, to purify the peptide. For the purification by chromatography, equipment such as high-performance liquid chromatography (HPLC) can be used.

### <Conjugate with IgG>

The present invention provides a conjugate in which the peptide vaccine combined with an IgG binding peptide is bound to IgG via an IgG binding peptide moiety (Figure 2). The IgG to be used in the present invention can be selected depending on the immune cells to which the peptide vaccine is intended to be delivered, and an IgG for the purpose of binging to the target immune cells, an IgG for binding to the target immune cells and activating the immune cells can be used in the present invention. For example, in the case of using dendritic cells as the immune cells, anti-CD40 antibody (IgG), anti-PD-L1 antibody (IgG), anti-DEC205 antibody (IgG), anti-DCIR antibody (IgG), anti-Mannose receptor antibody (IgG), anti-DC-SIGN antibody (IgG), anti-CD11c antibody (IgG), anti-Dectin-1 antibody (IgG) and the like can be used, but IgGs other than these antibodies also can be used depending on the purpose.

In the case of using vaccines against pathogens as peptide vaccines and in the case of using cancer vaccines as peptide vaccines, delivery to immune cells such as dendritic cells or B cells is effective. Accordingly, the IgG to be used in the present invention for delivery to such cells can be those known as IgGs against a target substance characteristic to the immune cells which delivers the peptide vaccine, and thus an antibody (clinically developed by J&J, Roche, Seattle Genetics, etc.) against CD40 as a target substance, an antibody (clinically developed by Celldex) against DEC205 as a target substance, an antibody (clinically developed by Ascend Biopharmaceuticals) against DCIR as a target substance, and the like can be used for delivery to dendritic cells.

The IgG binding peptide moiety and the IgG that can be used for forming the conjugate in the present invention can be made by modifying specific amino acid residues of the IgG binding peptide moiety of the present invention by a crosslinking agent and forming a crosslinked structure with the specific amino acid residues of IgG Fc via the crosslinking agent. The method for modifying the IgG binding peptide moiety of the peptide vaccine combined with an IgG binding peptide of the present invention by a crosslinking agent can be performed by peptide synthesis of the peptide vaccine combined with an IgG binding peptide using the amino acid residues modified by the crosslinking agent or synthesis of the peptide vaccine combined with an IgG binding peptide and subsequent modification specific to the side chain of specific amino acid residues in the IgG binding peptide moiety.

The crosslinking agent that can be used for forming the conjugate in the present invention can be appropriately selected by those skilled in the art, and a compound having at least two sites capable of binding to the specific amino acid residues of the IgG binding peptide moiety can be used therefor. Examples thereof can include, but not limited to, a crosslinking agent containing at least one, preferably, two or more succinimidyl groups such as disuccinimidyl glutarate (DSG) and disuccinimidyl suberate (DSS), a crosslinking agent containing at least one, preferably, two or more imide acid moieties such as dimethyl adipimidate dihydrochloride (dimethyl adipimidate-2HCl, DMA), dimethyl pimelimidate dihydrochloride (dimethyl pimelimidate-2HCl, DMP), or dimethyl suberimidate dihydrochloride (dimethyl suberimidate-2HCl, DMS), and a crosslinking agent having an SS bond in addition to the aforementioned functional group such as dimethyl 3,3'-dithio-bis(propionimidate) dihydrochloride (dimethyl 3,3'-dithiobispropionimidate-2HCl, DTBP) or dithiobis(succinimidylpropionate) (dithiobis(succinimidyl propionate), DSP) (WO2016/186206 (Patent Literature 1) and WO2018/230257 (Patent Literature 2)).

The specific amino acid residues by the crosslinking agent can be modified by selecting a combination of a crosslinking agent and specific amino acid residues (WO2016/186206 (Patent Literature 1) and WO2018/230257 (Patent Literature 2)). For example, in the case of using a crosslinking agent containing a succinimidyl group such as DSS or DSG, only the side chain of lysine residues disposed as the specific amino acid residues of the IgG binding peptide moiety can be specifically modified with DSS or DSG by blocking the N-terminal of the IgG binding peptide moiety and subsequent reaction with DSS or DSG, since the crosslinking agent reacts with the amine (ε amino group) in the side chain of lysine residues and the primary amine (alpha amino group) present at the N-terminal of the polypeptide. Such a combination of amino acid residues and a crosslinking agent can be appropriately selected by those skilled in the art. Alternatively, in the case of using DSS or DSG, in a peptide free from lysine residues, the primary amine (alpha amino group) present at the N-terminal of a polypeptide can be specifically modified by unmodifying the alpha amino group.

In the present invention, the peptide vaccine of the present invention in which the IgG binding peptide moiety is modified by a crosslinking agent can form a conjugate by mixing with the IgG, to bind to the IgG. The conditions for the mixing step are not specifically limited, as long as they are conditions in which a crosslinking reaction occurs between the IgG binding peptide moiety of the present invention modified by a crosslinking agent and the IgG. For example, the crosslinking reaction can be performed by mixing the peptide vaccine of the present invention containing the IgG binding peptide moiety modified by a crosslinking agent and the IgG in a suitable buffer at room temperature (e.g., about 15°C to 30°C). In the mixing step, an appropriate amount of a catalyst that promotes the crosslinking reaction may be added, as required.

At that time, in order to enhance the binding properties between the IgG binding peptide moiety and the IgG, other reaction conditions in the mixing step may be appropriately adjusted in consideration of the peptide vaccine of the present invention containing the IgG binding peptide moiety modified by a crosslinking agent and the type of the IgG. For example, conditions such as pH 4.5 to 6.5 (e.g., pH 5.0 to 6.0, pH 5.2 to 5.8, pH 5.4 to 5.6, or about pH 5.5) or pH 6.5 to 8.5 (e.g., pH 6.9 to 7.9, pH 7.2 to 7.7, pH 7.3 to 7.5, or about pH 7.4) can be selected as the pH at the time of reaction, the mixing ratio of the peptide vaccine of the present invention containing the IgG binding peptide moiety modified by a crosslinking agent to the IgG can be, for example, 1:1 to 20:1, 2:1 to 20:1, or 5:1 to 10:1, as a molar ratio, and the mixing time (reaction time) can be, for example, 1 minute to 5 hours, 10 minutes to 2 hours, or 15 minutes to 1 hour.

### <Use of conjugate>

According to another embodiment, the present invention can provide a method for delivering a peptide vaccine specifically to cells having target molecules targeted by the IgG on the cell surface using a conjugate of the peptide vaccine of the present invention combined with the aforementioned IgG binding peptide moiety and the IgG. Immune cells are selected as such desired cells, and the peptide vaccine is specifically delivered to the immune cells, thereby enabling an antibody against the peptide vaccine to be generated efficiently and cell-mediated immunity against the peptide vaccine such as activation of T cells against the peptide vaccine to be induced.

According to still another embodiment, the present invention can provide a vaccine composition for treating or preventing a target disease, comprising a conjugate of the peptide vaccine of the present invention combined with the aforementioned IgG binding peptide moiety and the IgG. The target disease to be treated or prevented by the vaccine composition of the present invention means the target from which the antigen of the peptide vaccine of the present invention is derived, and examples thereof can include infectious diseases against pathogens and cancers. Further, selection of an IgG depending on the immune cells which delivers the peptide vaccine enables an immune response to the immune cells to be induced. Accordingly, the vaccine composition of the present invention can be a vaccine composition for efficiently inducing generation of an antibody against the peptide vaccine or a vaccine composition for inducing cell-mediated immunity against the peptide vaccine such as activation of T cells against the peptide vaccine.

When used for treating a target disease, the vaccine composition of the present invention can comprise a peptide vaccine against the target disease and an IgG depending on the immune cells that induce the immune response intended to be induced in the patient who has developed the target disease. The target disease can be treated in the body of the patient by administering the vaccine composition of the present invention to the patient who has developed the target disease.

When used for preventing the target disease, the vaccine composition of the present invention can comprise a peptide vaccine against the target disease and an IgG depending on the immune cells that induce a preferred immune response intended to be induced against the target disease. An immune response when the cause of the target disease invades or occurs in the body of the subject can be induced in the body by administering the vaccine composition of the present invention to a subject who has not developed the target disease.

Since the delivery of the peptide vaccine of the present invention using an IgG to the immune cells is intended, the vaccine composition of the present invention can be parenterally administered by a route (such as intravenous injection, muscle injection, subcutaneous administration, or intraperitoneal administration) and can be prepared as various formulations in appropriate dosage forms depending on the administration route. The administration method and dosage form can be appropriately selected by those skilled in the art depending on the gender, age, body weight, symptoms, and the like of the patient.

The vaccine composition of the present invention can be formulated according to general knowledge in the art, including pharmaceutically acceptable carriers or additives. For example, when used as an injectable formulation, the conjugate of the present invention, for example, dissolved in a solution containing saline, a buffer, a glucose solution or the like and supplemented with a container adsorption inhibitor such as Tween 80, Tween 20, gelatin, human serum albumin can be used. Alternatively, it may be freeze-dried to form a dosage form to be dissolved for reconstruction before use, and examples of a stabilizer that can be used for freeze-drying include sugar alcohols such as mannitol and glucose and/or saccharides.

Hereinafter, the present invention will be specifically described with reference to examples. The examples shown below do not limit the present invention by any way.

### Examples

### Example 1: Production of anti-CD40 antibody

This example was performed to produce an agonist antibody specifically against CD40 as the surface antigen, for the purpose of selecting dendritic cells that induce vaccine-specific T cells as immune cells that deliver the peptide vaccine of the present invention and activating the cells.

### (1) Production of anti-mouse CD40 antibody

The anti-mouse CD40 antibody was produced as a chimeric antibody that fuses a variable region of FGK45 clone known as an anti-mouse CD40 agonist antibody with a constant region of a human antibody. The sequence (heavy chain: AEI27236.1 and light chain: AEI27235.1) published in NCBI (https://www.ncbi.nlm.nih.gov/) was used as the sequence of the variable region of FGK45 clone. A plasmid in which a light chain expression element (an EF-1alpha promoter, a secretion signal, a light-chain variable region, and a light-chain constant region were connected in tandem) and a heavy chain expression element (an EF-1alpha promoter, a secretion signal, a heavy-chain variable region, and a heavy-chain constant region were connected in tandem) were connected to pCI-neo was constructed as an expression vector.

The DNA sequences of the heavy-chain variable region and the light-chain variable region were designed based on the codons optimized for the hamster expression system based on the sequences obtained from the above database. The DNA sequences defining known amino acid sequences of the heavy-chain constant region moiety and the light-chain constant region moiety of a human antibody or the variants thereof can be used as the amino acid sequences of the heavy-chain constant region and the light-chain constant region. In this example, the sequences of a mutant (IgG1-lala) and human IgG kappa having L4A and L5A substitutions in the CH2 region of human IgG1 were used as the amino acid sequences of the heavy-chain constant region moiety and the light-chain constant region moiety, respectively.

ExpiCHO cells (invitrogen A2910002) were used as cells for antibody expression. The cells were cultured using ExpiCHO Expression Medium (Gibco, A2910002), and the antibody expression plasmid was transfected using ExpiCHO^{™} Expression System (GIbco A29129), followed by culture for 14 days, to collect a culture supernatant. The antibody was obtained by purifying the culture supernatant using a protein A column (MonoSpin ProA, GL Science 7510-11314).

The purity and binding properties of the purified antibody were confirmed based on SDS-PAGE and binding ability to mouse splenocytes, to confirm that the desired anti-mouse CD40 agonist antibody was obtained.

### (2) Production of anti-human CD40 antibody

The anti-human CD40 antibody was produced as a chimeric antibody fusing a variable region of 21.4.1 clone known as an anti-human CD40 agonist antibody and a constant region of a human antibody. The sequence published in a Patent Literature (Japanese Patent No. 4616555) was used as the sequence of 21.4.1 clone.

The production of the vector for the expression of the anti-human CD40 antibody and the expression/purification of the antibody were carried out by the same method as that used for the anti-mouse CD40 antibody described in (1).

The purity and binding properties of the purified antibody were confirmed based on SDS-PAGE and the target activation ability in CD40 forced expressing cells, to confirm that the desired anti-human CD40 agonist antibody was obtained.

### Example 2: Production of peptide

This example was performed to produce an antigenic peptide against each of various target substances as an example of the peptide vaccine of the present invention.

As the sequence of each peptide vaccine,
- a 16-mer peptide (EQLESIINFEKLTEWT [peptide (2)], SEQ ID NO: 29) containing an H-2Kb-restricted OVA-derived antigen peptide sequence(SIINFEKL [peptide (1), SEQ ID NO: 28]) whose antigen-specific T cell induction function in mice has been reported (Vaccine. 2004 Nov 25; 23(2): 258-66),
- a 16-mer peptide (RQYDPVAALFFFDIDL [peptide (6)], SEQ ID NO: 33) having a human A24:02-restricted CMV-derived antigen peptide sequence (PVAALFFF [peptide (5), SEQ ID NO: 32]) (J Transl Med. 2005 May 26; 3:23), or
- a 17-mer peptide (HLELASMTNMELMSSIV [peptide (9)], SEQ ID NO: 36) having an H-2Db-restricted neoantigen Adpgk-derived peptide sequence (ASMTNMELM [peptide (8), SEQ ID NO: 35]) (Nature. 2014 Nov 27; 515 (7528):572-6) was used.

As the IgG binding peptide sequence, a sequence (GPDCAYHRGELVWCTFH [IgG BP (1), (SEQ ID NO: 25)] or GPDCAYHKGELVWCTFH [IgG BP (2), (SEQ ID NO: 26)]) reported in Patent Literature PCT/JP2016/065061, and a newly created sequence (GPDCAWHRGELVWCTFH [IgG BP (3), (SEQ ID NO: 39)] or GPDCAWHLGELVWCTFH [IgG BP (4), (SEQ ID NO: 40)]) were used. In these sequences, the Xaa6 of the peptide described in SEQ ID NO: 4 is Arg, Lys, or Leu. Further, the sequence of an IgG binding peptide with partial mutation (GPDCAYHRGEAAACTFH [IgG BP (NC), SEQ ID NO: 27]) was used as the sequence of the IgG non-binding peptide as a negative control. These IgG binding peptides bind to the IgG antibody in a non-covalent bond to form a conjugate.

As a peptide for forming a conjugate (antibody-peptide vaccine conjugate (non-covalently bonded)) of the peptide vaccine and the antibody by a non-covalent bond, each peptide in which the vaccine peptide was connected in tandem to the N-terminal side of the IgG binding peptide, and two cysteines present in the IgG binding peptide were crosslinked by an S-S bond was synthesized [peptide (3-1) (SEQ ID NO: 30), peptide (7) (SEQ ID NO: 34), and (10) (SEQ ID NO: 37)] . Further, each peptide in which the vaccine peptide was connected in tandem to the N-terminal side of the IgG non-binding peptide ([IgG BP (NC) (SEQ ID NO: 27)]), and two cysteines present in the sequence of the IgG non-binding peptide were crosslinked by an SS bond was synthesized as a control peptide [peptide (4) (SEQ ID NO: 31) and peptide (11) (SEQ ID NO: 38)].

These peptides were produced by chemical synthesis. Further, a DBCO derivative peptide [peptide (6D)] in which a DBCO (Dibenzylcyclooctyne) group was imparted to the N-terminal side of the antigen peptide, and an IgG binding peptide derivative (IgG binding peptide-PEG-N3 [peptide (2P)]) in which a PEG linker and an azido group were imparted to the N-terminal side of the IgG binding peptide, and two cysteines were crosslinked by an SS bond were each produced as a peptide for forming an antibody-peptide vaccine conjugate (covalently bonded).

**[Table 1]**

| **Peptide No.** | **Sequence** | | | **SEQ ID NO** |
|---|---|---|---|---|
| **IgG BP(1)** | **GPDCAYHRGELVWCTFH** | | | **SEQ ID NO: 25** |
| **IgG BP(2)** | **GPDCAYHKGELVWCTFH** | | | **SEQ ID NO: 26** |
| **IgG BP(2P)** | **N3-PEG-GPDCAYHKGELVWCTFH** | | | |
| **IgG BP(3)** | **GPDCAWHRGELVWCTFH** | | | **SEQ ID NO: 39** |
| **IgG BP(4)** | **GPDCAWHLGELVWCTFH** | | | **SEQ ID NO: 40** |
| **IgG BP(NC)** | **GPDCAYHRGEAAACTFH** | | | **SEQ ID NO: 27** |
| **Peptide (1)** | | | **SIINFEKL** | **SEQ ID NO: 28** |
| **Peptide (2)** | | **EQLESIINFEKLTEWT** | | **SEQ ID NO: 29** |
| **Peptide (3-1)** | | **EQLESIINFEKLTEWT GPDCAYHRGELVWCTFH** | | **SEQ ID NO: 30 ((2)+IgG BP(1))** |
| **Peptide (3-2)** | | **EQLESIINFEKLTEWT GPDCAYHKGELVWCTFH** | | **SEQ ID NO: 41 ((2)+IgG BP(2))** |
| **Peptide (3-3)** | | **EQLESIINFEKLTEWT GPDCAWHRGELVWCTFH** | | **SEQ ID NO: 42 ((2)+IgG BP(3))** |
| **Peptide (3-4)** | | **EQLESIINFEKLTEWT GPDCAWHLGELVWCTFH** | | **SEQ ID NO: 43 ((2)+IgG BP(4))** |
| **Peptide (4)** | | **EQLESIINFEKLTEWT GPDCAYHRGEAAACTFH** | | **SEQ ID NO: 31 ((2)+IgG BP(NC))** |
| **Peptide (5)** | | | **PVAALFFF** | **SEQ ID NO: 32** |
| **Peptide (6)** | | **RQYDPVAALFFFDIDL** | | **SEQ ID NO: 33** |
| **Peptide (6D)** | **DBCO-RQYDPVAALFFFDIDL** | | | |
| **Peptide (7)** | | **RQYDPVAALFFFDIDL GPDCAYHKGELVWCTFH** | | **SEQ ID NO: 34 ((6)+ IqG BP(2))** |
| **Peptide (8)** | | | **ASMTNMELM** | **SEQ ID NO: 35** |
| **Peptide (9)** | | **HLELASMTNMELMSSIV** | | **SEQ ID NO: 36** |
| **Peptide (10)** | | **HLELASMTNMELMSSIV GPDCAYHRGELVWCTFH** | | **SEQ ID NO: 37 ((9)+ IgG BP(1))** |
| **Peptide (11)** | | **HLELASMTNMELMSSIV GPDCAYHRGEAAACTFH** | | **SEQ ID NO: 38 ((9)+IgG BP(NC))** |

### Example 3: Production of peptide vaccine-antibody conjugate

This example was performed to produce a conjugate of the peptide vaccine and the antibody. For producing the conjugate, a method of non-covalently complexing the antibody and the peptide vaccine and a method of covalently complexing them were used.

A conjugate of the peptide vaccine and the antibody by a non-covalent bond (antibody-peptide vaccine conjugate (non-covalently bonded)) was produced by mixing each peptide vaccine combined with the IgG binding peptide produced in Example 2 (peptide (3-1) (SEQ ID NO: 30), peptide (3-2) (SEQ ID NO: 41), peptide (3-3) (SEQ ID NO: 42), peptide (3-4) (SEQ ID NO: 43), peptide (7) (SEQ ID NO: 34), or peptide (10) (SEQ ID NO: 37)) and the anti-mouse CD40 antibody or the anti-human CD40 antibody produced in Example 1, or a commercially available human PD-L1 antibody (MedChemExpress CAS NO: 1380723-44-3) under conditions at room temperature.

A conjugate of the peptide vaccine and the antibody by a covalent bond (antibody-peptide vaccine conjugate (covalently bonded)) was produced as follows. First, a DBCO derivative peptide [peptide (6D)] and an IgG binding peptide-PEG-N3 [IgG BP (2P)] were prepared to 10 mM and 10.8 mM, respectively, using DMSO, followed by incubation for 2 hours by mixing at room temperature, to produce a fusion peptide of the two peptides (CMV-IgG binding peptide). Then, DSG (glutaric acid disuccinimidyl) dissolved in acetonitrile to a molar ratio of 1.4 times that of the CMV-IgG binding peptide was added thereto, followed by incubation for 4 hours at 50°C in the presence of pyridine, to produce a CMV-IgG binding peptide having the same sequence as peptide (7) and modified into succinimidyl glutarate.

The peptide was purified using HPLC and then mixed with the anti-human CD40 antibody or the isotype control antibody (human IgG1-lala antibody, autologously prepared) produced in Example 1 at a molar ratio of 10:1, followed by incubation for 3 hours at room temperature. Generation of an antibody-peptide vaccine conjugate (covalently bonded) was confirmed by electrophoresis, and then small molecules were removed by ultrafiltration.

### Example 4: Analysis of affinity of peptide for IgG

This example was performed for the purpose of examining the affinity for IgG of the H-2Kb-restricted OVA-derived peptide having each of the various IgG binding peptides prepared in Example 3.

Peptide (3-1) (SEQ ID NO: 30), peptide (3-2) (SEQ ID NO: 41), peptide (3-3) (SEQ ID NO: 42), peptide (3-4) (SEQ ID NO: 43), and peptide (4) (negative control, SEQ ID NO: 31) were used as the peptides to be evaluated.

For the H-2Kb restricted OVA-derived peptides having the aforementioned four different IgG binding peptide sequences, the binding activities to the antibody were evaluated. The IgG for which the affinity was evaluated was immobilized to a CM5 sensor chip (GE Health care) using the anti-CD40 antibody created in Example 1.

Specifically, the sample solution of each peptide was prepared by 3-fold dilution series from 3 nM to 243 nM, measured and analyzed with respect to the anti-CD40 antibody immobilized onto the CM5 sensor chip using BIAcore 8K (GE Health care) in the single kinetic mode. The dissociation constant was calculated using Biacore Insight Evaluation software to analyze the observation results.

As a result, it was confirmed that peptide (3-3) had about the same dissociation rate constant (Kd) as peptide (3-1) and had a higher affinity than peptide (3-2). In contrast, it was confirmed that peptide (3-4) had a higher affinity than peptide (3-2) and peptide (3-3). Further, peptide (4) as the negative control had a low affinity for the anti-CD40 antibody, and the dissociation rate constant and the affinity could not be calculated (Figure 3, Table 2).

**[Table 2]**

| **Peptide** | **Association rate constant (Ka)** | **Dissociation rate constant (Kd)** | **Affinity (KD)** |
|---|---|---|---|
| **Peptide (3-1)** | **3.31e+05** | **2.15e-02** | **6.49e-08** |
| **Peptide (3-2)** | **7.06e+04** | **1.21e-1** | **1.81e-6** |
| **Peptide (3-3)** | **2.75e+05** | **1.81e-02** | **6.58e-08** |
| **Peptide (3-4)** | **2.50e+05** | **4.44e-03** | **1.77e-08** |
| **Peptide (4)** | **N.D.** | **N.D.** | **N.D.** |

| | | | |
|---|---|---|---|
| **Note: N.D. indicates that calculation was not possible.** | | | |

### Example 5: In-vitro immunity induction test 1 (Whole PBMC method)

This example was performed for the purpose of examining whether or not the antibody-peptide vaccine conjugate (covalently bonded or non-covalently bonded) using each peptide vaccine produced in Example 3 had an ability to induce immunity in vitro.

Peripheral blood mononuclear cells (PBMCs) obtained from healthy volunteers were prepared to 2 × 10⁶ cells/mL using RPMI1640 medium (Thermo 61870-0362) containing 10% serum (BioWest, S4190) and seeded on a 24-well plate (FALCON, 353047) in 500 micro L each, followed by culture under conditions of 37°C and 5% CO₂.

The anti-CD40 antibody and peptide (7) (SEQ ID NO: 34) were respectively adjusted to 20 micro g/mL and 1 micro g/mL using RPMI1640 medium containing 10% serum and 200 U/mL IL-2 (NIPRO, 87890) and then added to the well plate on which the PBMCs had been seeded in 500 micro L each (to final concentrations of 10 micro g/mL and 0.5 micro g/mL, respectively) (group 2).

The antibody-peptide vaccine conjugate (covalently bonded) using the anti-CD40 antibody-CMV peptide was adjusted to 21 micro g/mL using RPMI1640 medium containing the same additives as group 2 above and then added to the well plate on which the PBMCs had been seeded in 500 micro L each (to a final concentration of 10.5 micro g/mL) (group 3).

As controls, the following groups were provided:
- the anti-CD40 antibody were adjusted to 20 micro g/mL using RPMI1640 medium containing 10% serum and 200 U/mL IL-2 (NIPRO, 87890) and then added to the well on which the PBMCs had been seeded in 500 micro L each (to a final concentration of 10 micro g/mL) (group 1);
- the isotype control antibody (human IgG1-lala antibody, autologously prepared) and peptide (7) respectively were adjusted to 20 micro g/mL and 1 micro g/mL using RPMI1640 medium containing 10% serum and 200 U/mL IL-2 (NIPRO, 87890) and then added to the well on which the PBMCs had been seeded in 500 micro L each (to final concentrations of 10 micro g/mL and 0.5 micro g/mL, respectively) (group 4); and
- the antibody-peptide vaccine conjugate (covalently bonded) of the isotype control antibody and the DSG peptide produced in Example 3 was adjusted to 21 micro g/mL using RPMI1640 medium containing the same additives as group 2 above and then added to the well on which the PBMCs had been seeded in 500 micro L each (to a final concentration of 10.5 micro g/mL) (group 5).

LPS (SIGMA, L2762), R848 (Invivogen, vac-r848), and poly I:C (Invivogen, tlrl-pic) were respectively adjusted to 800 ng/mL, 2 mM, and 4 micro g/mL using RPMI1640 medium containing 10% serum and 200 U/mL IL-2 and then added to the well plate on which the PBMCs had been seeded in 330 micro L each.

After culture under conditions of 37°C and 5% CO₂ for 7 days, the cells were collected and stained using APC-CMV-tetramer (MBL, TS-0020-2C), BV421-anti-human CD3 antibody (Biolegend,300434), and FITC-anti-human CD8 antibody (Biolegend, 300906), followed by FACS analysis.

Figure 4 shows the results. Peptide-specific T cell induction was evaluated based on the proportion of tetramer-positive cells in CD3 and CD8-positive cells. As a result, both non-covalently bonded (group 2) and covalently bonded (group 3) anti-CD40 antibody-peptide vaccine conjugates efficiently induced vaccine-specific T cells as compared with the isotype control antibody vaccine conjugates (group 4 and group 5) (Figure 4).

### Example 6: In-vitro immunity induction test 2 (Monocyte-derived dendritic cell (MDDC) method)

This example was performed for the purpose of examining whether or not the antibody-peptide vaccine conjugate (covalently bonded or non-covalently bonded) using each peptide vaccine produced in Example 3 had an ability to induce immunity in vitro by another method from that in Example 5.

CD14-positive monocytes were purified from PBMCs obtained from healthy volunteers using CD14 MACS beads (Miltenyl Biotec, 130-050-20110). CD14-negative cells were separately cryopreserved using a cell cryopreservation solution (Takara, CB011).

The CD14-positive monocytes purified were prepared to 1 × 10⁶ cells/mL using RPMI1640 medium (Thermo, 61870-036) containing 10% FBS (SIGMA, 172072-500ML), 10 mM HEPES (nacalai, 17557-94), 50 micro M 2-mercapto ethanol (2-ME; nacalai, 21438-82), 6.5 ng/mL IL-4 (peprotech, 200-04), and 100 ng/mL GM-CSF (R&D, 215-GM-500), seeded on a 24-well plate (FALCON, 353047) in 1 mL each, followed by culture under conditions of 37°C and 5% CO₂.

The cells were collected 6 days after the start of culture, adjusted to 6 × 10⁴ cells/mL using RPMI1640 containing 10% FBS (SIGMA, 172072-500ML), non-essential amino acid (nacalai, 06344-56), 1 mM Sodium Pyruvate (nacalai, 06977-34), 10 mM HEPES (nacalai, 17557-94), and 50 micro M 2ME (nacalai, 21438-82), and seeded on a 96-well U-bottom plate (FALCON, 353077) in 50 micro L each.

The anti-human CD40 antibody and peptide (7) (SEQ ID NO: 34) were respectively adjusted to 40 micro g/mL and 2 micro g/mL using the same medium as used above for culturing the monocytes and then added to the 96 well plate on which the CD14-positive monocytes had been seeded in 50 micro L each (to final concentrations of respectively 10 micro g/mL and 0.5 micro g/mL) (group 2).

The antibody-peptide vaccine conjugate (covalently bonded) by the anti-CD40 antibody-CMV peptide was adjusted to 42 micro g/mL using RPMI1640 medium containing the same additives as group 2 above and then added to the 96 well plate on which the CD14-positive monocytes had been seeded in 50 micro L each (to a final concentration of 10.5 micro g/mL) (group 3).

As controls, the following groups were prepared:
- the anti-human CD40 antibody were adjusted to 40 micro g/mL using RPMI1640 medium containing 10% serum and 200 U/mL IL-2 (NIPRO, 87890) and then added to the 96 well plate on which the CD14-positive monocytes had been seeded in 50 micro L each (to a final concentration of 10 micro g/mL) (group 1);
- the isotype control antibody (human IgG1-lala antibody, autologously prepared) and peptide (7) were respectively adjusted to 40 microg/mL and 2 micro g/mL using RPMI1640 medium containing 10% serum and 200 U/mL IL-2 (NIPRO, 87890) and then added to the 96 well plate on which the CD14-positive monocytes had been seeded in 50 micro L each (to final concentrations of 10 micro g/mL and 0.5 micro g/mL, respectively) (group 4); and
- the antibody-peptide vaccine conjugate (covalently bonded) by the isotype control antibody and the DSG peptide produced in Example 3 was adjusted to 42 micro g/mL using RPMI1640 medium containing the same additives as group 2 above and then added to the 96 well plate on which the CD14-positive monocytes had been seeded in 50 micro L each (to a final concentration of 10.5 micro g/mL) (group 5).

Further, LPS, R848, poly I:C, and IL-2 were respectively adjusted to 800 ng/mL, 2 mM, 4 micro g/mL, and 80 U/mL using the medium and then added in 50 micro L each, in the same manner as in Example 5.

7 days from the start of culture under conditions of 37°C and 5% CO₂, the frozen CD14-negative cells were recovered and purified into CD8-positive cells using CD8 MACS beads (Miltenyl Biotec, 130-045-201) . The cells were adjusted to 1.8 × 10⁶ cells/mL using RPMI1640 containing 10% FBS, non-essential amino acid, 1 mM Sodium Pyruvate, 10 mM HEPES and 50 micro M 2ME and added in 50 micro L each, followed by culture at 37°C. The cells were collected 14 days from the start of the test and stained using APC-CMV-tetramer (MBL, TS-0020-2C), BV421-anti-CD3 antibody (Biolegend,300434), and FITC-anti-CD8 antibody (Biolegend, 300906), followed by FACS analysis.

Figure 5 shows the results. Peptide-specific T cell induction was evaluated based on the proportion of tetramer-positive cells in CD3 and CD8-positive cells. As a result, the anti-CD40 antibody vaccine conjugates (both non-covalently bonded (group 2) and covalently bonded (group 3)) efficiently induced vaccine-specific T cells as compared with the isotype control antibody vaccine conjugates (group 4 and group 5) (Figure 5).

### Example 7: Evaluation of peptide uptake into dendritic cells in wild-type mice

This example was performed for the purpose of examining whether or not the antibody-peptide conjugate using each peptide vaccine produced in Example 3, when subcutaneously administered, was taken up by the dendritic cells (classical dendritic cell 1/2, (cDC1/cDC2)) present in the lymph nodes in the vicinity of the administration site, in vivo.

In this example, IgG BP (1) peptide (SEQ ID NO: 25) (the entire sequence: K*RRK*RRK*RRGPDCAYHRGELVWCTFH) supplemented with a peptide (K*RRK*RRK*RR) ("K*" indicating FAM-binding lysine) labeled with the fluorescent dye FAM (6-Carboxyfluorescein) was used as a test peptide.

7 week-old, female, wild-type C57BL/6 mice (CLEA Japan, Inc.) were divided into three groups, with three mice per group, and experiments were performed with the group composition in Table 3. Specifically, to each group, the following was subcutaneously administered to the inguinal region:
- group 1: only the adjuvant PolyIC:LC (available from Oncovir, Inc.);
- group 2: a mixture of the fluorescently labeled IgG BP (1) peptide (test peptide) and the adjuvant PolyIC:LC (available from Oncovir, Inc.); and
- group 3: a mixture of the antibody-peptide vaccine conjugate (non-covalently bonded) by the test peptide and the anti-mouse CD40 antibody, with the adjuvant PolyIC:LC (available from Oncovir, Inc.) further added.

The dosage of each ingredient is as shown in Table 3.

### [Table 3]

**Table 3: Group composition of mice**

| **Administration group** | | **Test peptide** | **Anti-CD40 antibody** | **PolyIC:LC** |
|---|---|---|---|---|
| **1** | **Adjuvant (Negative control)** | **-** | **-** | **8 micro g** |
| **2** | **Peptide vaccine +Adjuvant** | **11.7 micro g** | **-** | **8 micro g** |
| **3** | **Antibody-vaccine conjugate +Adjuvant** | **11.7 micro g** | **200 micro g** | **8 micro g** |

Administration was performed by administering a single dose of the drug to each group of mice. The inguinal lymph nodes in the vicinity of the administration site were collected 5 hours later, and the tissue was disrupted to prepare lymph node cells. Thereafter, the cells were stained with the anti-CD11c antibody, the anti-I-A/I-E antibody, the anti-XCR1 antibody, and the anti-CD172a antibody, followed by flow cytometry analysis.

The uptake of fluorescently labeled IgG BP (1) peptide was evaluated based on the proportion of the FAM-positive cells in cDC1 cells (CD11c+/I-A/I-E+/XCR1+/CD172-) and cDC2 cells (CD11c+/I-A/I-E+/XCR1-/CD172+).

Figure 6 shows the results. As a result of comparing group 1 with group 2 and group 1 with group 3, the peptide uptake into both the cDC1 cells and the cDC2 cells was promoted more in the antibody-peptide vaccine conjugate (non-covalently bonded)-administered group (group 3) than in the group in which the peptide vaccine was administered alone without antibodies (group 2) (Figure 6).

### Example 8: Immunity induction test 1 in wild-type mice (comparative test with conventional vaccine regimen)

This example was performed for the purpose of examining whether or not the antibody-peptide vaccine conjugate (non-covalently bonded) using each peptide vaccine produced in Example 3 had an ability to induce immunity, in vivo.

7 week-old, female, wild-type C57BL/6 mice (CLEA Japan, Inc.) were divided into five groups, with six mice per group, and experiments were performed with the group composition in Table 4. Specifically, to each group, the following was administered:
- group 1: only the peptide vaccine (the H-2Kb-restricted OVA-derived peptide (3-1) of Example 2, SEQ ID NO: 30);
- group 2: a mixture of peptide (3-1) and the adjuvant PolyIC:LC (available from Oncovir, Inc.);
- group 3: the antibody-peptide vaccine conjugate (non-covalently bonded) by peptide (3-1) and the anti-mouse CD40 antibody;
- group 4: a mixture of the antibody-peptide vaccine conjugate (non-covalently bonded) by peptide (3-1) and the anti-mouse CD40 antibody, with the adjuvant PolyIC:LC (available from Oncovir, Inc.) further added; and
- group 5: a negative control free from any of peptide (3-1), the anti-mouse CD40 antibody, and the adjuvant.
The dosage of each ingredient is as shown in Table 4.

The administration was performed according to the schedule shown in Figure 7. Specifically, each drug was subcutaneously administered to the mice of each group every 7 days twice in total (on day 0 and day 7 when the first administration day was taken as day 0). The spleen was collected on day 14, the tissue was disrupted to prepare the splenocytes, and the cells were stained with the anti-CD8 antibody and PE-OVA Tetramer (MBL, TS-5001-1C), followed by flow cytometry analysis. Induction of Ova specific T cells was evaluated based on the proportion of tetramer-positive cells in the CD8-positive T cells.

### [Table 4]

**Table 4: Group composition of mice**

| **Administration group** | | **Peptide (3-1)** | **Anti-CD40 antibody** | **PolyIC:LC** |
|---|---|---|---|---|
| **1** | **Peptide vaccine** | **21.1 micro g** | **-** | **-** |
| **2** | **Peptide vaccine +Adjuvant** | **21.1 micro g** | **-** | **8 micro g** |
| **3** | **Antibody-vaccine conjugate** | **21.1 micro g** | **400 micro g** | **-** |
| **4** | **Antibody-vaccine conjugate +Adjuvant** | **21.1 micro g** | **400 micro g** | **8 micro g** |
| **5** | **Control** | **-** | **-** | **-** |

Figure 8 shows the results. As a result of comparing group 1 with group 2 and group 3 with group 4, an ability to promote T cell induction was exhibited in the presence of the adjuvant PolyIC:LC (group 1 and group 2, and group 3 and group 4) (Figure 8). In the presence of the adjuvant PolyIC:LC, as a result of comparing group 2 with group 4, an ability to promote T cell induction was exhibited even in the group in which the peptide vaccine was administered alone without antibodies (group 2), but a more significant ability to promote T cell induction was exhibited in the antibody-peptide vaccine conjugate (non-covalently bonded)-administered group (group 4) than in group 2 (group 2 and group 4) (Figure 8).

### Example 9: Immunity induction test 2 in wild-type mice (comparison test for antibody-vaccine mixing ratio)

This example was performed for the purpose of examining the effect of the mixing ratio of the antibody and each peptide vaccine produced in Example 3 in the antibody-peptide vaccine conjugate (non-covalently bonded) using the peptide vaccine on the ability to induce immunity, in vivo.

7 week-old, female, wild-type C57BL/6 mice (CLEA Japan, Inc.) were divided into groups each with four mice, and experiments were performed with the group composition in Table 5. Specifically, to each group, the following was administered:
- group 1: a mixture of the antibody-peptide vaccine conjugate (non-covalently bonded) by two mole equivalents of the peptide vaccine (the H-2Kb-restricted OVA-derived peptide (3-1) of Example 2, SEQ ID NO: 30) and one mole equivalent of the anti-mouse CD40 antibody, with the adjuvant PolyIC:LC (available from Oncovir, Inc.) further added;
- group 2: a mixture of two mole equivalents of the IgG non-binding peptide (peptide (4), SEQ ID NO: 31) as a control peptide, one mole equivalent of the anti-mouse CD40 antibody, and the adjuvant PolyIC:LC (available from Oncovir, Inc.);
- group 3: a mixture of the antibody-peptide vaccine conjugate (non-covalently bonded) by four mole equivalents of peptide (3-1) and one mole equivalent of the anti-mouse CD40 antibody, with the adjuvant PolyIC:LC (available from Oncovir, Inc.) further added; and
- group 4: a mixture of four mole equivalents of the IgG non-binding peptide (peptide (4)) as a control peptide, one mole equivalent of the anti-mouse CD40 antibody, and the adjuvant PolyIC:LC (available from Oncovir, Inc.). The dosage of each ingredient is as shown in Table 5.

The administration was performed according to the schedule shown in Figure 7. Specifically, each drug was subcutaneously administered to the mice of each group every 7 days twice in total (on day 0 and day 7 when the first administration day was taken as day 0). The spleen was collected on day 14, and induction of Ova specific T cells was evaluated by the same method as in Example 8.

### [Table 5]

**Table 5: Group composition of mice**

| **Administration group** | | **Ova-IgG binding Peptide [Peptide (3-1)]** | **Ova-IgG non-binding peptide [Peptide (4)]** | **Anti-CD40 antibody** | **PolyIC:LC** |
|---|---|---|---|---|---|
| **1** | **Antibody: Ova-IgG binding peptide two equal-dose group** | **10.5 micro g** | **-** | **200 micro 9** | **8 micro g** |
| **2** | **Antibody: Ova-IgG non-binding peptide two equal-dose group** | **-** | **10 micro g** | **200 micro 9** | **8 micro g** |
| **3** | **Antibody: Ova-IgG binding peptide four equal-dose group** | **21.1 micro g** | **-** | **200 micro 9** | **8 micro g** |
| **4** | **Antibody: Ova-IgG non-binding peptide four equal-dose group** | **-** | **20.1 micro g** | **200 micro 9** | **8 micro g** |

Figure 9 shows the results. When comparing the group of the antibody-peptide vaccine conjugate (non-covalently bonded) (anti-CD40 antibody: Ova-IgG binding peptide) with the group of a mixture of the antibody and the Ova-IgG non-binding peptide, the efficiency of immunity induction in the conjugate was enhanced in both the two mole equivalents-group (group 1 and group 2) and the four mole equivalents-group (group 3 and group 4) (Figure 9). It can be seen from these results that, when the two mole equivalents-groups were compared with the four mole equivalents-groups, the four mole equivalents-groups showed stronger immunity induction in all of the groups of the antibody and the Ova-IgG binding peptide (group 1 and group 3) and the groups of the antibody and the Ova-IgG non-binding peptide (group 2 and group 4). It was demonstrated that the effect of induction by the peptide excessively added in the four mole equivalents-groups was added.

### Example 10: Immunity induction test 3 in wild-type mice (dose-dependent test)

This example was performed for the purpose of examining the dose dependency of the antibody-peptide vaccine conjugate (non-covalently bonded) using each peptide vaccine produced in Example 3, in vivo.

7 week-old, female, wild-type C57BL/6 mice (CLEA Japan, Inc.) were divided into groups each with four mice, and experiments were performed with the group composition in Table 6. Specifically, groups 1 to 4 had a high dose of the peptide vaccine (10.5 micro g of the peptide vaccine) administered, and groups 5 to 8 had a medium dose of the peptide vaccine (2.1 micro g of the peptide vaccine) administered. Specifically, to each group, the following was administered:
- group 1: a mixture of a high dose (10.5 micro g) of the peptide vaccine (the H-2Kb-restricted OVA-derived peptide (3-1) of Example 2, SEQ ID NO: 30) and the adjuvant PolyIC:LC (available from Oncovir, Inc.);
- group 2: a mixture of the antibody-peptide vaccine conjugate (non-covalently bonded) by a high dose (10.5 micro g, equivalent to the two mole equivalents) of the peptide vaccine (peptide (3-1) of Example 2) and the anti-mouse CD40 antibody (200 micro g, equivalent to the one mole equivalent) and the adjuvant PolyIC:LC (available from Oncovir, Inc.);
- group 3: a mixture of a high dose (10.0 micro g) of the IgG non-binding peptide (peptide (4), SEQ ID NO: 31) as a control peptide and the adjuvant PolyIC:LC (available from Oncovir, Inc.);
- group 4: a mixture of a high dose (10.0 micro g, equivalent to the two mole equivalents) of the IgG non-binding peptide (peptide (4)) as a control peptide, the anti-mouse CD40 antibody (200 micro g, equivalent to the one mole equivalent) as an anti-mouse CD40 antibody, and the adjuvant PolyIC:LC (available from Oncovir, Inc.);
- group 5: a mixture of a medium dose (2.1 micro g) of the peptide vaccine (peptide (3-1) of Example 2) and the adjuvant PolyIC:LC (available from Oncovir, Inc.);
- group 6: a mixture of the antibody-peptide vaccine conjugate (non-covalently bonded) by a medium dose (2.1 micro g, equivalent to the two mole equivalents) of the peptide vaccine (peptide (3-1) of Example 2) and the anti-mouse CD40 antibody (40 micro g, equivalent to the one mole equivalent) and the adjuvant PolyIC:LC (available from Oncovir, Inc.);
- group 7: a mixture of a medium dose (2.0 micro g) of the IgG non-binding peptide (peptide (4)) as a control peptide and the adjuvant PolyIC:LC (available from Oncovir, Inc.); and
- group 8: a mixture of a medium dose (2.0 micro g, equivalent to the two mole equivalents) of the IgG non-binding peptide (peptide (4)) as a control peptide, the anti-mouse CD40 antibody (40 micro g, equivalent to the one mole equivalent) as an anti-mouse CD40 antibody, and the adjuvant PolyIC:LC (available from Oncovir, Inc.). The dosage of each ingredient is as shown in Table 6.

The administration was performed according to the schedule shown in Figure 7. Specifically, each drug was subcutaneously administered to the mice of each group every 7 days twice in total (on day 0 and day 7 when the first administration day was taken as day 0). The spleen was collected on day 14, by the same method as in Example 8 induction of Ova specifically T cells was evaluated.

### [Table 6]

**Table 6: Group composition of mice**

| **Administration group** | | **Ova-IgG binding peptide [Peptide (3-1)]** | **Ova-IgG non-binding peptide [Peptide (4)]** | **Anti-CD40 antibody** | **PolyIC:LC** |
|---|---|---|---|---|---|
| **1** | **Ova-IgG binding peptide high-dose group** | **10.5 micro g** | **-** | **-** | **8 micro g** |
| **2** | **Antibody: Ova-IgG binding peptide high-dose group** | **10.5 micro g** | **-** | **200 micro 9** | **8 micro g** |
| **3** | **Ova-IgG non-binding peptide high-dose group** | **-** | **10 micro g** | **-** | **8 micro g** |
| **4** | **Antibody: Ova-IgG non-binding peptide high-dose group** | **-** | **10 micro g** | **200 micro 9** | **8 micro g** |
| **5** | **Ova-IgG binding peptide medium-dose group** | **2.1 micro g** | **-** | **-** | **8 micro g** |
| **6** | **Antibody: Ova-IgG binding peptide medium-dose group** | **2.1 micro g** | **-** | **40 micro 9** | **8 micro g** |
| **7** | **Ova-IgG non-binding peptide medium-dose group** | **-** | **2.0 micro g** | **-** | **8 micro g** |
| **8** | **Antibody: Ova-IgG non-binding peptide medium-dose group** | **-** | **2.0 micro g** | **40 micro 9** | **8 micro g** |

Figure 10 shows the results. The antibody: Ova-IgG binding peptide groups (groups 2 and 6) showed a higher immunity induction effect than the Ova-IgG binding peptide groups (groups 1 and 5), the Ova-IgG non-binding peptide groups (groups 3 and 7), and the antibody: Ova-IgG non-binding peptide groups (groups 4 and 8). Further, the effect was dose-dependent (Figure 10).

### Example 11: Cancer-bearing mouse test 1 (evaluation of antibody-peptide vaccine conjugate: conjugate with anti-CD40 antibody)

This example was performed for the purpose of examining whether or not the antibody-peptide vaccine conjugate (non-covalently bonded) produced using each peptide vaccine produced in Example 3 and combined with the anti-CD40 antibody had an anti-tumor effect, in vivo.

E.G7 (ATCC (R)CRL-2113) as a mouse T cell lymphoma cell line forced to express the Ova antigen was subcutaneously transplanted into 7 week-old, female, wild-type C57BL/6 mice (CLEA Japan, Inc.) at 1 × 10⁶ cells per mouse. The transplantation day was taken as day 0, and the mice were grouped (six mice per group) based on the tumor volume on day 3. Experiments were performed on each group with the group composition in Table 7. Specifically, to each group, the following was subcutaneously administered:
- group 1: only saline (negative control);
- group 2: the adjuvant PolyIC:LC (available from Oncovir, Inc.) (negative control);
- group 3: a mixture of peptide (3-1) (SEQ ID NO: 30) and the adjuvant PolyIC:LC (available from Oncovir, Inc.);
- group 4: a mixture of the antibody-peptide vaccine conjugate (non-covalently bonded) by peptide (3-1) and the anti-mouse CD40 antibody and the adjuvant PolyIC:LC (available from Oncovir, Inc.);
- group 5: a mixture of the IgG non-binding peptide (peptide (4), SEQ ID NO: 31) as a control peptide and the adjuvant PolyIC:LC (available from Oncovir, Inc.); and
- group 6: a mixture of the IgG non-binding peptide (peptide (4)) as a control peptide, the anti-mouse CD40 antibody, and the adjuvant PolyIC:LC (available from Oncovir, Inc.). The amount of each inoculation is as shown in Table 7.

The drug was subcutaneously administered to the mice of each group twice in total on day 3 and day 10, and the tumor volume and the body weight were observed at a frequency of twice a week from the start of the drug administration.

### [Table 7]

**Table 7: Group composition of mice**

| **Administration group** | | **Ova-IgG binding peptide [Peptide (3-1)]** | **Ova-IgG non-binding peptide [Peptide (4)]** | **Anti-CD40 antibody** | **PolyIC:LC** |
|---|---|---|---|---|---|
| **1** | **Saline group** | **-** | **-** | **-** | **-** |
| **2** | **Poly IC:LC group** | **-** | **-** | **-** | **8 micro g** |
| **3** | **Ova-IgG binding peptide** | **2.1 micro g** | **-** | **-** | **8 micro g** |
| **4** | **Antibody: Ova-IgG binding peptide** | **2.1 micro g** | **-** | **40 micro g** | **8 micro g** |
| **5** | **Ova-IgG non-binding peptide** | **-** | **2 micro g** | **-** | **8 micro g** |
| **6** | **Antibody: Ova-IgG non-binding peptide** | **-** | **2 micro g** | **40 micro g** | **8 micro g** |

Figure 11 and Figure 12 show the results. It was demonstrated that the antibody: Ova-IgG binding peptide group (group 4) showed a higher tumor growth inhibitory effect in vivo than the Ova-IgG binding peptide group (group 3), the Ova-IgG non-binding peptide group (group 5), and the antibody: Ova-IgG non-binding peptide group (group 6) (Figure 11).

As the survival period of each individual mouse was investigated, it was confirmed that the antibody: Ova-IgG binding peptide group (group 4) showed a more remarkable prolonging effect on the survival period than the Ova-IgG binding peptide group (group 3), the Ova-IgG non-binding peptide group (group 5), and the antibody: Ova-IgG non-binding peptide group (group 6) (Figure 12) .

### Example 12: Immunity induction test 4 in wild-type mice (neoantigen model)

This example was performed for the purpose of evaluating whether or not the antibody-peptide vaccine conjugate (non-covalently bonded) using each peptide vaccine produced in Example 3 had an anti-tumor effect in a test system with higher clinical extrapolation, specifically, a test system employing a peptide sequence determined based on the mutation specific to cancer as a vaccine sequence.

7 week-old, female, wild-type C57BL/6 mice (CLEA Japan, Inc.) were divided into groups each with four mice, and experiments were performed with the group composition in Table 8. Specifically, to each group, the following was administered:
- group 1: a mixture of the peptide vaccine (peptide (10) of Example 2, SEQ ID NO: 37) and the adjuvant PolyIC:LC (available from Oncovir, Inc.);
- group 2: a mixture of the antibody-peptide vaccine conjugate (non-covalently bonded) by the peptide vaccine (peptide (10) of Example 2) and the anti-mouse CD40 antibody and the adjuvant PolyIC:LC (available from Oncovir, Inc.);
- group 3: a mixture of the IgG non-binding peptide (peptide (11), SEQ ID NO: 38) as a control peptide and the adjuvant PolyIC:LC (available from Oncovir, Inc.);
- group 4: a mixture of the IgG non-binding peptide (peptide (11)) as a control peptide, the anti-mouse CD40 antibody as an anti-mouse CD40 antibody, and the adjuvant PolyIC:LC (available from Oncovir, Inc.); and
- group 5: only the adjuvant PolyIC:LC. The dosage of each ingredient is as shown in Table 8.

### [Table 8]

**Table 8: Group composition of mice**

| **Administration group** | | **Adpgk-IgG binding peptide [Peptide (10)]** | **Adpgk-IgG non-binding peptide [Peptide (11)]** | **Anti-CD40 antibody** | **PolyIC:LC** |
|---|---|---|---|---|---|
| **1** | **Adpgk-IgG binding peptide group** | **10.5 micro g** | **-** | **-** | **8 micro g** |
| **2** | **Antibody: Adpgk-IgG binding peptide group** | **10.5 micro g** | **-** | **200 micro 9** | **8 micro g** |
| **3** | **Adpgk-IgG non-binding peptide group** | **-** | **10 micro g** | **-** | **8 micro g** |
| **4** | **Antibody: Adpgk-IgG non-binding peptide group** | **-** | **10 micro g** | **200 micro 9** | **8 micro g** |
| **5** | **Saline group** | | | | **8 micro g** |

The administration was performed according to the schedule shown in Figure 13. Specifically, each drug was subcutaneously administered to the mice of each group every 4 days three times in total (on day 0, day 4 and day 8 when the first administration day was taken as day 0).

The spleen was collected on day 15, then 3 × 10⁶ cells were suspended in 100 micro L of Splenocyte growth medium (RPMI-1640 (Thermo 61870-0362) containing 10% FBS (BioWest, S4190), 1 mM sodium pyruvate (Wako, 190-14881), 0.05 mM 2-ME (Wako, 131-14572), 20 mM HEPES (Wako, 345-06681), and 1% penicillin streptomycin (nakarai, 26253-84)) and seeded, and 100 micro L of a solution in which peptide (8) (SEQ ID NO: 35) was dissolved was added thereto to a final concentration of 10 micro g/mL, followed by culture under conditions of 37°C and 5% CO₂ for 15 hours.

22 micro L of 10 × Brefeldin A (Biolegend 420601) was added, followed by incubation for 3 to 4 hours and subsequent centrifugation at 2000 rpm, and the cells were collected and stained with Zombie Violet (BioLegend 423114), APC anti-mouse CD4 (BioLegend 100412), FITC anti-mouse CD8a (BioLegend 100706), and PE anti-mouse IFN-g (BioLegend 505807), followed by flow cytometry analysis. Immunity activation against Adpgk peptide was evaluated based on the proportion of the IFNgamma-positive cells in CD8-positive cells.

Figure 14 shows the results. The antibody: Adpgk-IgG binding peptide group (group 2) showed a higher immunity induction effect than the Adpgk-IgG binding peptide group (group 1), the Adpgk-IgG non-binding peptide group (group 3), and the antibody: Adpgk-IgG non-binding peptide group (group 4). In this way, since the antibody-binding neoantigen (group 2) showed the highest immunity induction, the usefulness of the antibody vaccine was suggested also in this model.

### Example 13: Immunity induction test 5 in wild-type mice (evaluation of plurality of antibody-vaccine peptides)

This example was performed for the purpose of examining whether or not the antibody-peptide vaccine conjugate (non-covalently bonded) using each peptide vaccine produced in Example 3 and combined with the CD40 antibody or the PD-L1 antibody had an ability to induce immunity, in vivo.

In this example, 7 week-old, female, wild-type C57BL/6 mice (CLEA Japan, Inc.) were first divided into four groups with five mice per group, and experiments were performed with the group composition in Table 9. Specifically,
- group 1: only the adjuvant PolyIC:LC (available from Oncovir, Inc.);
- group 2: a mixture of the peptide vaccine (the H-2Kb-restricted OVA-derived peptide (3-1) of Example 2, SEQ ID NO: 30) and the adjuvant PolyIC:LC (available from Oncovir, Inc.);
- group 3: a mixture of the antibody-peptide vaccine conjugate (non-covalently bonded) by peptide (3-1) and the anti-mouse CD40 antibody, with the adjuvant PolyIC:LC (available from Oncovir, Inc.) further added; and
- group 4: a mixture of the antibody-peptide vaccine conjugate (non-covalent bond) by peptide (3-1) and the anti-PD-L1 antibody, with the adjuvant PolyIC:LC (available from Oncovir, Inc.) further added
were each administered. In this example, human PD-L1 antibody (MedChemExpress CAS NO: 1380723-44-3) was used as a PD-L1 antibody. The dosage of each ingredient is as shown in Table 9.

### [Table 9]

**Table 9: Group composition of mice**

| **Administration group** | | **Peptide (3-1)** | **Antibody** | **PolyIC:LC** |
|---|---|---|---|---|
| **1** | **Adjuvant (Negative control)** | **-** | **-** | **8 micro g** |
| **2** | **Peptide vaccine +Adjuvant** | **10.5 micro g** | **-** | **8 micro g** |
| **3** | **Antibody-vaccine conjugate +Adjuvant** | **10.5 micro g** | **Anti-CD40 antibody 200 micro g** | **8 micro g** |
| **4** | **Antibody-vaccine conjugate +Adjuvant** | **10.5 micro g** | **Anti-PD-L1 antibody 200 micro g** | **8 micro g** |

The administration was performed according to the schedule shown in Figure 15. Specifically, each drug was subcutaneously administered to the mice of each group every 7 days twice in total (on day 0 and day 7 when the first administration day was taken as day 0). The spleen was collected on day 14, by the same method as in Example 8, then Ova-specific T cell induction was evaluated.

Figure 16 shows the results. As a result of comparing group 2 with group 3 and group 2 with group 4, an ability to promote T cell induction was shown even in the group in which the peptide vaccine was administered together with the adjuvant without antibodies (group 2), but a more remarkable ability to promote T cell induction was shown in the PD-L1 antibody-peptide vaccine non-covalent conjugate-administered group (group 4), as in the CD40 antibody-peptide vaccine non-covalent bond conjugate-administered group (group 3), than in group 2 (group 3 and group 4) (Figure 16).

### Example 14: Immunity induction test 6 in wild-type mice (evaluation of plurality of antibody-vaccine peptides)

This example was performed, as in Example 13, for the purpose of examining whether or not the antibody-peptide vaccine conjugate (non-covalently bonded) using each peptide vaccine produced in Example 3 and combined with the CD40 antibody or the PD-L1 antibody, when administered with a peptide vaccine and an antibody that do not form a conjugate, had an ability to promote immunity induction, in vivo.

7 week-old, female, wild-type C57BL/6 mice (CLEA Japan, Inc.) were divided into five groups, with six mice per group, and experiments were performed with the group composition in Table 10. Specifically,
- group 1: only the adjuvant PolyIC:LC (available from Oncovir, Inc.);
- group 2: a mixture of the IgG non-binding peptide (peptide (4), SEQ ID NO: 31) as a control peptide and the anti-mouse CD40 antibody, with the adjuvant PolyIC:LC (available from Oncovir, Inc.) further added;
- group 3: a mixture of the antibody-peptide vaccine conjugate (non-covalently bonded) by peptide (3-1) and the anti-mouse CD40 antibody, with the adjuvant PolyIC:LC (available from Oncovir, Inc.) further added;
- group 4: a mixture of peptide (4) and the anti-PD-L1 antibody, with the adjuvant PolyIC:LC (available from Oncovir, Inc.) further added; and
- group 5: a mixture of the antibody-peptide vaccine conjugate (non-covalently bonded) by peptide (3-1) and the anti-PD-L1 antibody, with the adjuvant PolyIC:LC (available from Oncovir, Inc.) further added
were each administered. In this example, human PD-L1 antibody (MedChemExpress CAS NO: 1380723-44-3) was used as a PD-L1 antibody. The dosage of each ingredient is as shown in Table 10.

### [Table 10]

**Table 10: Group composition of mice**

| **Administration group** | | **Peptide (3-1)** | **Peptide (4)** | **Antibody** | **PolyIC:LC** |
|---|---|---|---|---|---|
| **1** | **Adjuvant (Negative control)** | **-** | **-** | **-** | **8 micro g** |
| **2** | **Antibody + Peptide vaccine +Adjuvant** | **-** | **10.5 micro g** | **Anti-CD40 antibody 200 micro g** | **8 micro g** |
| **3** | **Antibody-vaccine conjugate +Adjuvant** | **10.5 micro g** | **-** | **Anti-CD40 antibody 200 micro g** | **8 micro g** |
| **4** | **Antibody + Peptide vaccine + Adjuvant** | **-** | **10.5 micro g** | **Anti-PD-L1 antibody 200 micro g** | **8 micro g** |
| **5** | **Antibody-vaccine conjugate + Adjuvant** | **10.5 micro g** | **-** | **Anti-PD-L1 antibody 200 micro g** | **8 micro g** |

The administration was performed according to the schedule shown in Figure 15, as in Example 13. Specifically, each drug was subcutaneously administered to the mice of each group every 7 days twice in total (on day 0 and day 7 when the first administration day was taken as day 0). The spleen was collected on day 14, and induction of Ova-specific T cells was evaluated by the same method as in Example 8.

Figure 17 shows the results. As a result of comparing group 2 with group 3 and group 4 with group 5, T cell induction was exhibited even in the groups in which the peptide vaccine without binding to antibodies was administered together with the antibodies (group 2 and group 4), but T cell induction was promoted more remarkably in the CD40 antibody-peptide vaccine conjugate (non-covalently bonded)-administered group (group 3) or the PD-L1 the antibody-peptide vaccine conjugate (non-covalently bonded)-administered group (group 5) (Figure 17).

### Example 15: Cancer-bearing mouse test 2 (evaluation of antibody-peptide vaccine conjugate: conjugate with anti-PD-L1 antibody)

This example was performed for the purpose of examining whether or not the antibody-peptide vaccine conjugate (non-covalently bonded) produced using each peptide vaccine produced in Example 3 and combined with an anti-PD-L1 antibody had an anti-tumor effect, in vivo.

E.G7 (ATCC (R)CRL-2113) as a mouse T cell lymphoma cell line forced to express the Ova antigen was subcutaneously transplanted into 7 week-old, female, wild-type C57BL/6 mice (CLEA Japan, Inc.) at 1 × 10⁶ cells per mouse. The transplantation day was taken as day 0, and the mice were grouped (six mice per group) based on the tumor volume on day 4. Experiments were performed on each group with the group composition in Table 11. Specifically,
- group 1: the adjuvant PolyIC:LC (available from Oncovir, Inc.) (negative control);
- group 2: a mixture of peptide (3-1) (SEQ ID NO: 30) and the adjuvant PolyICLC (available from Oncovir, Inc.);
- group 3: a mixture of the anti-PD-L1 antibody alone and adjuvant PolyICLC (available from Oncovir, Inc.) (control); and
- group 4: a mixture of the antibody-peptide vaccine conjugate (non-covalently bonded) by peptide (3-1) and the anti-PD-L1 antibody and the adjuvant PolyIC:LC (available from Oncovir, Inc.) were each subcutaneously administered to each group. In this example, human PD-L1 antibody (MedChemExpress CAS NO: 1380723-44-3) was used as a PD-L1 antibody. The amount of each inoculation is as shown in Table 11.

The drug was subcutaneously administered to the mice of each group twice in total on day 4 and day 11, and the tumor volume and the body weight were observed at a frequency of twice a week from the start of the drug administration.

### [Table 11]

**Table 11: Group composition of mice**

| **Administration group** | | **Peptide (3-1)** | **Anti-PD-L1 antibody** | **PolyIC:LC** |
|---|---|---|---|---|
| **1** | **Adjuvant (Negative control)** | **-** | **-** | **8 micro g** |
| **2** | **Peptide vaccine +Adjuvant** | **2.1 micro g** | **-** | **8 micro g** |
| **3** | **Antibody +Adjuvant** | **-** | **40 micro g** | **8 micro g** |
| **4** | **Antibody-vaccine conjugate +Adjuvant** | **2.1 micro g** | **40 micro g** | **8 micro g** |

Figure 18 and Figure 19 show the results. It was demonstrated that the antibody: Ova-IgG binding peptide group (group 4) showed a higher tumor growth inhibitory effect in vivo than the Ova-IgG binding peptide group (group 2) and the anti-PD-L1 antibody alone group (group 3) (Figure 18).

As the survival period of each individual mouse was investigated, it was confirmed that the antibody: Ova-IgG binding peptide group (group 4) showed a more remarkable prolonging effect on the survival period than the Ova-IgG binding peptide group (group 2) and the anti-PD-L1 antibody alone group (group 3) (Figure 19).

### Example 16: Immunity induction test 7 in wild-type mice (neoantigen model)

This example was performed for the purpose of examining whether or not the antibody-peptide vaccine conjugate (non-covalently bonded) containing the non-synonymous somatic mutation sequence of a mouse tumor cell line had an anti-tumor effect, in vivo.

First, peptides each containing a non-synonymous somatic mutation sequence (each 27-mer, 30 peptides of peptide (101) to peptide (130)) were created as the non-synonymous somatic mutation sequence of a mouse tumor cell line, as shown in Table 12 below, based on the analysis results of the genome sequence obtained from the mouse tumor cell line MC-38.

### [Table 12]

**Table 12: Non-synonymous somatic cell mutation sequence of mouse tumor cell line**

| **Peptide** | **Sequence** | **SEQ ID NO:** |
|---|---|---|
| **Peptide (101)** | **KLAEGAERDGKNNVAFMSYFLQGTLDA** | **SEQ ID NO: 44** |
| **Peptide (102)** | **LLQFEVCPLSESRFSLSFQHPVNDSLV** | **SEQ ID NO: 45** |
| **Peptide (103)** | **TEPIYQPHSAWVPFGGLMGLALLGALL** | **SEQ ID NO: 46** |
| **Peptide (104)** | **PPRALPPVPSAIQANIPLSHPKKKKSR** | **SEQ ID NO: 47** |
| **Peptide (105)** | **AHDSSKDSVTVFVNNLPYSIEEPEVKL** | **SEQ ID NO: 48** |
| **Peptide (106)** | **RTPKLSSIIDCTGTASYNGFLPVLVRN** | **SEQ ID NO: 49** |
| **Peptide (107)** | **IILGKSHYVLYGLIAAMQPRMLVTFDE** | **SEQ ID NO: 50** |
| **Peptide (108)** | **HQVRVYDPVSPQHRPVLEATYGEYPLE** | **SEQ ID NO: 51** |
| **Peptide (109)** | **TSSGPGVTSAVDFKLHIEVLHYLLQSW** | **SEQ ID NO: 52** |
| **Peptide (110)** | **EASEEGEEEEDEEDLEAMQSRLPTLRS** | **SEQ ID NO: 53** |
| **Peptide (111)** | **LEIDHYVGSAPPLLEGPLPSRNSVPIL** | **SEQ ID NO: 54** |
| **Peptide (112)** | **NEQDSLWLDVTASLKIRNSNFYPVAV** | **SEQ ID NO: 55** |
| **Peptide (113)** | **GIPVHLELASMTNMELMSSIVHQVFPT** | **SEQ ID NO: 56** |
| **Peptide (114)** | **LLPTEQRMLMKSVLTRLRTPMNLPKLM** | **SEQ ID NO: 57** |
| **Peptide (115)** | **MTPELQEKMVSFRSIFKDLEDKKVNQQ** | **SEQ ID NO: 58** |
| **Peptide (116)** | **TRTSSSSLTSSVPVAPQLEEAPLTVKS** | **SEQ ID NO: 59** |
| **Peptide (117)** | **GPDIHRIQEKPRNNRVAVATINFRRLV** | **SEQ ID NO: 60** |
| **Peptide (118)** | **DDDETGKISFKNLTRVAKELGENLTDE** | **SEQ ID NO: 61** |
| **Peptide (119)** | **HESGHAIIAYYTKAAMPINKATIMPRG** | **SEQ ID NO: 62** |
| **Peptide (120)** | **KRNENCTLQFEAALALTNIASGTSQQT** | **SEQ ID NO: 63** |
| **Peptide (121)** | **EALAQKTYSDDHVTFFCFQVLEHQVKY** | **SEQ ID NO: 64** |
| **Peptide (122)** | **GHGHWVNTMALSTYYALRTGAFEPAEA** | **SEQ ID NO: 65** |
| **Peptide (123)** | **YPFYSFCDVKQYIEHSILRVAETGPEH** | **SEQ ID NO: 66** |
| **Peptide (124)** | **AKMKGYPHWPALIDDIADGAVKPPPID** | **SEQ ID NO: 67** |
| **Peptide (125)** | **NSRLTADLSAQLQNLTSTESQLKEVRS** | **SEQ ID NO: 68** |
| **Peptide (126)** | **EVGCRGPEASPVSPGEPLRRASRSGGR** | **SEQ ID NO: 69** |
| **Peptide (127)** | **WTVTLYPSSPVTNYRWRSFTGAVTLEK** | **SEQ ID NO: 70** |
| **Peptide (128)** | **ASSLIAHVRQHTGRSPTSVNAVARDLS** | **SEQ ID NO: 71** |
| **Peptide (129)** | **LWISTRIWALWSWVSWNQDPFLKASSL** | **SEQ ID NO: 72** |
| **Peptide (130)** | **RDGFLGPPNPPFLWALRIRITLPFPQP** | **SEQ ID NO: 73** |

Thereafter, the IgG BP (1) (SEQ ID NO: 25) was added to the aforementioned peptides (27-mer, peptide (101) to peptide (130)), to create peptides (each 44mer, 30 peptides of peptide (101BP) to peptide (130BP)) as shown in Table 13 below.

### [Table 13]

**Table 13: IgG binding peptide based on non-synonymous somatic cell mutation sequence of mouse tumor cell line**

| **IgG binding peptide** | **Sequence** |
|---|---|
| **Peptide (101BP)** | **KLAEGAERDGKNNVAFMSYFLQGTLDA-IgG BP(1)** |
| **Peptide (102BP)** | **LLQFEVCPLSESRFSLSFQHPVNDSLV-IgG BP(1)** |
| **Peptide (103BP)** | **TEPIYQPHSAWVPFGGLMGLALLGALL-IgG BP(1)** |
| **Peptide (104BP)** | **PPRALPPVPSAIQANIPLSHPKKKKSR-IgG BP(1)** |
| **Peptide (105BP)** | **AHDSSKDSVTVFVNNLPYSIEEPEVKL-IgG BP(1)** |
| **Peptide (106BP)** | **RTPKLSSIIDCTGTASYNGFLPVLVRN-IgG BP(1)** |
| **Peptide (107BP)** | **IILGKSHYVLYGLIAAMQPRM LVTFDE-IgG BP(1)** |
| **Peptide (108BP)** | **HQVRVYDPVSPQHRPVLEATYGEYPLE-IgG BP(1)** |
| **Peptide (109BP)** | **TSSGPGVTSAVDFKLHIEVLHYLLQSW-IgG BP(1)** |
| **Peptide (110BP)** | **EASEEGEEEEDEEDLEAMQSRLPTLRS-IgG BP(1)** |
| **Peptide (111BP)** | **LEIDHYVGSAPPLLEGPLPSRNSVPIL-IgG BP(1)** |
| **Peptide (112BP)** | **NEQDSLVVLDVTASLKIRNSNFYPVAV-IgG BP(1)** |
| **Peptide (113BP)** | **GIPVHLELASMTNMELMSSIVHQVFPT-IgG BP(1)** |
| **Peptide (114BP)** | **LLPTEQRMLMKSVLTRLRTPMNLPKLM-IgG BP(1)** |
| **Peptide (115BP)** | **MTPELQEKMVSFRSIFKDLEDKKVNQQ-IgG BP(1)** |
| **Peptide (116BP)** | **TRTSSSSLTSSVPVAPQLEEAPLTVKS-IgG BP(1)** |
| **Peptide (117BP)** | **GPDIHRIQEKPRNNRVAVATINFRRLV-IgG BP(1)** |
| **Peptide (118BP)** | **DDDETGKISFKNLTRVAKELGENLTDE-IgG BP(1)** |
| **Peptide (119BP)** | **HESGHAIIAYYTKAAMPINKATIMPRG-IgG BP(1)** |
| **Peptide (120BP)** | **KRNENCTLQFEAALALTNIASGTSQQT-IgG BP(1)** |
| **Peptide (121BP)** | **EALAQKTYSDDHVTFFCFQVLEHQVKY-IgG BP(1)** |
| **Peptide (122BP)** | **GHGHWVNTMALSTYYALRTGAFEPAEA-IgG BP(1)** |
| **Peptide (123BP)** | **YPFYSFCDVKQYIEHSILRVAETGPEH-IgG BP(1)** |
| **Peptide (124BP)** | **AKMKGYPHWPALIDDIADGAVKPPPID-IgG BP(1)** |
| **Peptide (125BP)** | **NSRLTADLSAQLQNLTSTESQLKEVRS-IgG BP(1)** |
| **Peptide (126BP)** | **EVGCRGPEASPVSPGEPLRRASRSGGR-IgG BP(1)** |
| **Peptide (127BP)** | **WTVTLYPSSPVTNYRWRSFTGAVTLEK-IgG BP(1)** |
| **Peptide (128BP)** | **ASSLIAHVRQHTGRSPTSVNAVARDLS-IgG BP(1)** |
| **Peptide (129BP)** | **LWISTRIWALWSWVSWNQDPFLKASSL-IgG BP(1)** |
| **Peptide (130BP)** | **RDGFLGPPNPPFLWALRIRITLPFPQP-IgG BP(1)** |

| | |
|---|---|
| **Note: IgG BP(1): GPDCAYHRGELVWCTFH(SEQ ID NO: 25)** | |

7 week-old, female, wild-type C57BL/6 mice (CLEA Japan, Inc.) were divided into two groups, and experiments were performed with the group composition in Table 14. Specifically, to two mice per pool, the following was subcutaneously administered:
- group 1: a mixture of each six vaccine peptides (e.g., pool 01 (peptide (101) to peptide (106)) to pool 06 (peptide (125) to peptide (130))) pooled from the peptide vaccines shown in Table 12 above (each 27-mer, peptide (101) to peptide (130)) and the adjuvant PolyIC:LC (available from Oncovir, Inc.); and
- group 2: a mixture of the antibody-peptide vaccine conjugate (non-covalently bonded) (e.g., pool 11 (peptide (101BP) to peptide (103BP)) to pool 20 (peptide (128BP) to peptide (130BP))) by each three peptides pooled from the IgG binding peptides (each 44mer, peptide (101BP) to peptide (130BP)) shown in Table 13 above, antibody-peptide vaccine conjugate (non-covalently bonded) complexing the anti-mouse CD40 antibody and the adjuvant PolyIC:LC (available from Oncovir, Inc.). The amount of each inoculation is as shown in Table 14.

### [Table 14]

**Table 14: Group composition of mice**

| **Administration group** | | **Peptide** | **Anti-CD40 antibody** | **PolyIC:LC** |
|---|---|---|---|---|
| **1** | **Peptide vaccine +Adjuvant** | **5 micro g/peptide ×6 types/Pool** | **(-)** | **16 micro g** |
| **2** | **Antibody-vaccine conjugate + Adjuvant** | **5 micro g/peptide ×3 types/Pool** | **200 micro g** | **16 micro g** |

The administration was performed according to the schedule shown in Figure 20. Specifically, each drug was subcutaneously administered to the mice of each group every 7 days three times in total (on day 0, day 7, and day 14 when the first administration day was taken as day 0). The spleen was collected on day 21, then 2 × 10⁶ cells were suspended in 100 micro L of Splenocyte growth medium (RPMI-1640 (Thermo 61870-0362) containing 10% FBS (BioWest, S4190), 1 mM sodium pyruvate (Wako, 190-14881), 0.05 mM 2-ME (Wako, 131-14572), 20 mM HEPES (Wako, 345-06681), and 1% penicillin streptomycin (Nakarai, 26253-84)) and seeded on a filter plate (Millipore) coated with a mouse IFN-gamma antibody (MabTech), and 100 micro L of a solution in which peptide (101e) to peptide (130e)) (SEQ ID NO: 74-SEQ ID NO: 103) that are the respective epitope peptides of the peptides shown in Table 15 were individually dissolved to a final concentration of 10 micro g/mL was added thereto, followed by culture under conditions of 37°C and 5% CO₂ for 15 hours.

### [Table 15]

**Table 15: Epitope peptide sequence of each peptide**

| **Epitope peptide** | **Sequence** | **SEQ ID NO** |
|---|---|---|
| **Peptide (101e)** | **MSYFLQGTL** | **SEQ ID NO: 74** |
| **Peptide (102e)** | **FSLSFQHPV** | **SEQ ID NO: 75** |
| **Peptide (103e)** | **SAWVPFGGL** | **SEQ ID NO: 76** |
| **Peptide (104e)** | **SAIQANIPL** | **SEQ ID NO: 77** |
| **Peptide (105e)** | **SVTVFVNNL** | **SEQ ID NO: 78** |
| **Peptide (106e)** | **ASYNGFLPV** | **SEQ ID NO: 79** |
| **Peptide (107e)** | **VLYGLIAAM** | **SEQ ID NO: 80** |
| **Peptide (108e)** | **VSPQHRPVL** | **SEQ ID NO: 81** |
| **Peptide (109e)** | **SAVDFKLHI** | **SEQ ID NO: 82** |
| **Peptide (110e)** | **AMQSRLPTL** | **SEQ ID NO: 83** |
| **Peptide (111e)** | **SAPPLLEGPL** | **SEQ ID NO: 84** |
| **Peptide (112e)** | **WLDVTASL** | **SEQ ID NO: 85** |
| **Peptide (113e)** | **ASMTNMELM** | **SEQ ID NO: 86** |
| **Peptide (114e)** | **SVLTRLRTPM** | **SEQ ID NO: 87** |
| **Peptide (115e)** | **VSFRSIFKDL** | **SEQ ID NO: 88** |
| **Peptide (116e)** | **SSLTSSVPV** | **SEQ ID NO: 89** |
| **Peptide (117e)** | **VATINFRRL** | **SEQ ID NO: 90** |
| **Peptide (118e)** | **ISFKNLTRV** | **SEQ ID NO: 91** |
| **Peptide (119e)** | **AAMPINKATI** | **SEQ ID NO: 92** |
| **Peptide (120e)** | **CTLQFEAAL** | **SEQ ID NO: 93** |
| **Peptide (121e)** | **VTFFCFQVL** | **SEQ ID NO: 94** |
| **Peptide (122e)** | **MALSTYYAL** | **SEQ ID NO: 95** |
| **Peptide (123e)** | **YSFCDVKQYI** | **SEQ ID NO: 96** |
| **Peptide (124e)** | **KGYPHWPAL** | **SEQ ID NO: 97** |
| **Peptide (125e)** | **AQLQNLTST** | **SEQ ID NO: 98** |
| **Peptide (126e)** | **ASPVSPGEPL** | **SEQ ID NO: 99** |
| **Peptide (127e)** | **RSFTGAVTL** | **SEQ ID NO: 100** |
| **Peptide (128e)** | **RSPTSVNAV** | **SEQ ID NO: 101** |
| **Peptide (129e)** | **VSWNQDPFL** | **SEQ ID NO: 102** |
| **Peptide (130e)** | **WALRIRITL** | **SEQ ID NO: 103** |

The splenocytes and each peptide added were removed by aspiration, the filter plate was further washed, and then 100 micro L of the biotin-labeled mouse IFN-gamma antibody (MabTech) was added thereto, followed by incubation at 37°C for 2 hours. The filter plate was washed, and 100 micro L of Streptavidin-ALP (MabTech) was added thereto, followed by further incubation for 1 hour. The filter plate was washed again, 100 micro L of a substrate solution (MabTech; BCIP/NBT-plus) was added thereto, and IFNgamma secreted by the cells was detected.

Figure 21 shows the results. Comparing with the 27-mer peptide vaccine group (group 1), it was confirmed that the group in which the antibody-peptide vaccine conjugate (non-covalently bonded) by the anti-mouse CD40 antibody was administered (group 2) had a stronger immune response against the same epitope sequence.

### Industrial Applicability

The peptide vaccine combined with an IgG binding peptide disclosed in the present invention enables the peptide vaccine to be efficiently delivered to the surface of specific immune cells (e.g., dendritic cells) and enhance the activation to enhance the effects of the peptide vaccine.

## Claims

1. A peptide vaccine combined with an IgG binding peptide.

2. The peptide vaccine according to claim 1, wherein
the peptide vaccine is selected from the group consisting of a cancer vaccine and an infectious disease vaccine.

3. The peptide vaccine according to claim 2, wherein
the peptide vaccine is a cancer vaccine selected from the group consisting of a cancer-associated antigen, a cancer neoantigen, and a cancer personalized neoantigen.

4. The peptide vaccine according to any one of claims 1 to 3, wherein
the IgG binding peptide is selected from the group consisting of:
Xaa1 Xaa2 Xaa3 Cys Xaa4 Xaa5 His Xaa6 Gly Xaa7 Leu Val Trp Cys Xaa8 Xaa9 Xaa10 (SEQ ID NO: 1)
wherein
each of Xaa1, Xaa2, Xaa9, and Xaa10 is any amino acid other than Cys or is absent, or Xaa1 and Xaa2 are combined to form Arg Arg Gly Pro,
each of Xaa3, Xaa4, Xaa5, and Xaa8 is any amino acid other than Cys,
Xaa6 is Lys, Cys, Asp, Glu, Arg, Leu, 2-amino suberic acid, or diaminopropionic acid, and
Xaa7 is Glu, Asn, or Gln; and
Xaa11 Xaa12 Asn Met Gln Cys Gln Arg Arg Phe Tyr Glu Ala Leu His Asp Pro Asn Leu Asn Glu Glu Gln Arg Asn Ala Xaa13 Ile Xaa14 Ser Ile Arg Asp Asp Cys (SEQ ID NO: 2)
wherein
Xaa11 is selected from the group consisting of Gly, Ala, Val, Leu, Ile, Met, Pro, Phe, Trp, Lys, ornithine, Cys, Asp, Glu, beta-Ala, 2-amino suberic acid, diaminopropionic acid, and NH₂-(PEG)n-CO (n = 1 to 50) or is absent,
Xaa12 is selected from the group consisting of Lys, ornithine, Cys, Asp, Glu, Phe, 2-amino suberic acid, and diaminopropionic acid, and
Xaa13 and Xaa14 are each independently selected from the group consisting of Arg, His, Asp, Glu, Ser, Thr, Asn, Gln, Tyr, and Cys.

5. The peptide vaccine according to any one of claims 1 to 4, wherein
Xaa4 is selected from the group consisting of Ala, Ser, and Thr, and
Xaa5 is Tyr or Trp.

6. The peptide vaccine according to claim 4, wherein
the IgG binding peptide is selected from the group consisting of:
Asp Cys Ala Tyr His Xaa6 Gly Glu Leu Val Trp Cys Thr (SEQ ID NO: 3) 
Gly Pro Asp Cys Ala Tyr His Xaa6 Gly Glu Leu Val Trp Cys Thr
Phe His (SEQ ID NO: 4)
 Arg Cys Ala Tyr His Xaa6 Gly Glu Leu Val Trp Cys Ser (SEQ ID NO: 5) 
Gly Pro Arg Cys Ala Tyr His Xaa6 Gly Glu Leu Val Trp Cys Ser
Phe His (SEQ ID NO: 6)
 
Ser Pro Asp Cys Ala Tyr His Xaa6 Gly Glu Leu Val Trp Cys Thr
Phe His (SEQ ID NO: 7)
 
Gly Asp Asp Cys Ala Tyr His Xaa6 Gly Glu Leu Val Trp Cys Thr
Phe His (SEQ ID NO: 8)
 
Gly Pro Ser Cys Ala Tyr His Xaa6 Gly Glu Leu Val Trp Cys Thr
Phe His (SEQ ID NO: 9)
 
Gly Pro Asp Cys Ala Tyr His Xaa6 Gly Glu Leu Val Trp Cys Ser
Phe His (SEQ ID NO: 10)
 
Gly Pro Asp Cys Ala Tyr His Xaa6 Gly Glu Leu Val Trp Cys Thr
His His (SEQ ID NO: 11)
 
Gly Pro Asp Cys Ala Tyr His Xaa6 Gly Glu Leu Val Trp Cys Thr
Phe Tyr (SEQ ID NO: 12)
 
Ser Pro Asp Cys Ala Tyr His Xaa6 Gly Glu Leu Val Trp Cys Thr
Phe Tyr (SEQ ID NO: 13)
 
Ser Asp Asp Cys Ala Tyr His Xaa6 Gly Glu Leu Val Trp Cys Thr
Phe Tyr (SEQ ID NO: 14)
 
Arg Gly Asn Cys Ala Tyr His Xaa6 Gly Gln Leu Val Trp Cys Thr
Tyr His (SEQ ID NO: 15)
 
Gly Xaa2 Asp Cys Ala Tyr His Xaa6 Gly Glu Leu Val Trp Cys Thr
Xaa9 His (SEQ ID NO: 16)
 
Arg Arg Gly Pro Asp Cys Ala Tyr His Xaa6 Gly Glu Leu Val Trp
Cys Thr Phe His (SEQ ID NO: 17)
 
Phe Asn Met Gln Cys Gln Arg Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 18),
 
Gly Phe Asn Met Gln Cys Gln Arg Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 19),
 
Lys Asn Met Gln Cys Gln Arg Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 20),
 
Gly Phe Asn Met Gln Cys Gln Lys Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 21),
 
Lys Asn Met Gln Cys Gln Lys Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 22),
 
Phe Asn Met Gln Gln Gln Arg Arg Phe Tyr Glu Ala Leu His Asp
Pro Asn Leu Asn Glu Glu Gln Arg Asn Ala Arg Ile Arg Ser Ile Arg
Asp Asp (SEQ ID NO: 23); and
 and

Gly Lys Asn Met Gln Cys Gln Arg Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 24).

7. The peptide vaccine according to claim 6, wherein
the IgG binding peptide is 
Gly Pro Asp Cys Ala Tyr His Arg Gly Glu Leu Val Trp Cys Thr
Phe His (SEQ ID NO: 25);
 
Gly Pro Asp Cys Ala Tyr His Lys Gly Glu Leu Val Trp Cys Thr
Phe His (SEQ ID NO: 26);
 
Gly Pro Asp Cys Ala Trp His Arg Gly Glu Leu Val Trp Cys Thr
Phe His (SEQ ID NO: 39); or
 or

Gly Pro Asp Cys Ala Trp His Leu Gly Glu Leu Val Trp Cys Thr
Phe His (SEQ ID NO: 40).

8. The peptide vaccine according to any one of claims 1 to 7, wherein
an IgG is bound to the IgG binding peptide.

9. The peptide vaccine according to claim 8, wherein
the IgG is an antibody against a target substance characteristic to immune cells which delivers the peptide vaccine.

10. The peptide vaccine according to claim 9, wherein
the immune cells are selected from the group consisting of antigen-presenting cells, dendritic cells, and B cells.

11. The peptide vaccine according to any one of claims 8 to 10, wherein
the IgG is selected from the group consisting of an anti-CD40 antibody, an anti-PD-L1 antibody, an anti-DEC205 antibody, an anti-DCIR antibody, an anti-Mannose receptor antibody, an anti-DC-SIGN antibody, an anti-CD11c antibody, and an anti-Dectin-1 antibody.

12. A method of delivering a peptide vaccine to cells having molecules targeted by the IgG on the cell surface by using an IgG, an IgG binding peptide, and a peptide vaccine combined with the IgG binding peptide.

13. The method according to claim 12, wherein
the peptide vaccine is selected from the group consisting of a cancer vaccine and an infectious disease vaccine.

14. The method according to claim 13, wherein
the peptide vaccine is a cancer vaccine selected from the group consisting of a cancer-associated antigen, a cancer neoantigen, and a cancer personalized neoantigen.

15. The method according to any one of claims 12 to 14, wherein the IgG binding peptide is selected from the group consisting of:
Xaa1 Xaa2 Xaa3 Cys Xaa4 Xaa5 His Xaa6 Gly Xaa7 Leu Val Trp Cys Xaa8 Xaa9 Xaa10 (SEQ ID NO: 1)
wherein
each of Xaa1, Xaa2, Xaa9, and Xaa10 is any amino acid other than Cys or is absent, or Xaa1 and Xaa2 are combined to form Arg Arg Gly Pro,
each of Xaa3, Xaa4, Xaa5, and Xaa8 is any amino acid other than Cys,
Xaa6 is Lys, Cys, Asp, Glu, Arg, Leu, 2-amino suberic acid, or diaminopropionic acid, and
Xaa7 is Glu, Asn, or Gln; and
Xaa11 Xaa12 Asn Met Gln Cys Gln Arg Arg Phe Tyr Glu Ala Leu His Asp Pro Asn Leu Asn Glu Glu Gln Arg Asn Ala Xaa13 Ile Xaa14 Ser Ile Arg Asp Asp Cys (SEQ ID NO: 2)
wherein
Xaa11 is selected from the group consisting of Gly, Ala, Val, Leu, Ile, Met, Pro, Phe, Trp, Lys, ornithine, Cys, Asp, Glu, beta-Ala, 2-amino suberic acid, diaminopropionic acid, and NH2-(PEG)n-CO (n = 1 to 50) or is absent,
Xaa12 is selected from the group consisting of Lys, ornithine, Cys, Asp, Glu, Phe, 2-amino suberic acid, and diaminopropionic acid, and
Xaa13 and Xaa14 are each independently selected from the group consisting of Arg, His, Asp, Glu, Ser, Thr, Asn, Gln, Tyr, and Cys.

16. The method according to any one of claims 12 to 15, wherein
Xaa4 is selected from the group consisting of Ala, Ser, and Thr, and
Xaa5 is Tyr or Trp.

17. The method according to claim 15, wherein
the IgG binding peptide is selected from the group consisting of:
Asp Cys Ala Tyr His Xaa6 Gly Glu Leu Val Trp Cys Thr (SEQ ID NO: 3) 
Gly Pro Asp Cys Ala Tyr His Xaa6 Gly Glu Leu Val Trp Cys Thr
Phe His (SEQ ID NO: 4)
 Arg Cys Ala Tyr His Xaa6 Gly Glu Leu Val Trp Cys Ser (SEQ ID NO: 5) 
Gly Pro Arg Cys Ala Tyr His Xaa6 Gly Glu Leu Val Trp Cys Ser
Phe His (SEQ ID NO: 6)
 
Ser Pro Asp Cys Ala Tyr His Xaa6 Gly Glu Leu Val Trp Cys Thr
Phe His (SEQ ID NO: 7)
 
Gly Asp Asp Cys Ala Tyr His Xaa6 Gly Glu Leu Val Trp Cys Thr
Phe His (SEQ ID NO: 8)
 
Gly Pro Ser Cys Ala Tyr His Xaa6 Gly Glu Leu Val Trp Cys Thr
Phe His (SEQ ID NO: 9)
 
Gly Pro Asp Cys Ala Tyr His Xaa6 Gly Glu Leu Val Trp Cys Ser
Phe His (SEQ ID NO: 10)
 
Gly Pro Asp Cys Ala Tyr His Xaa6 Gly Glu Leu Val Trp Cys Thr
His His (SEQ ID NO: 11)
 
Gly Pro Asp Cys Ala Tyr His Xaa6 Gly Glu Leu Val Trp Cys Thr
Phe Tyr (SEQ ID NO: 12)
 
Ser Pro Asp Cys Ala Tyr His Xaa6 Gly Glu Leu Val Trp Cys Thr
Phe Tyr (SEQ ID NO: 13)
 
Ser Asp Asp Cys Ala Tyr His Xaa6 Gly Glu Leu Val Trp Cys Thr
Phe Tyr (SEQ ID NO: 14)
 
Arg Gly Asn Cys Ala Tyr His Xaa6 Gly Gln Leu Val Trp Cys Thr
Tyr His (SEQ ID NO: 15)
 
Gly Xaa2 Asp Cys Ala Tyr His Xaa6 Gly Glu Leu Val Trp Cys Thr
Xaa9 His (SEQ ID NO: 16)
 
Arg Arg Gly Pro Asp Cys Ala Tyr His Xaa6 Gly Glu Leu Val Trp
Cys Thr Phe His (SEQ ID NO: 17)
 
Phe Asn Met Gln Cys Gln Arg Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 18),
 
Gly Phe Asn Met Gln Cys Gln Arg Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 19),
 
Lys Asn Met Gln Cys Gln Arg Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 20),
 
Gly Phe Asn Met Gln Cys Gln Lys Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 21),
 
Lys Asn Met Gln Cys Gln Lys Arg Phe Tyr Glu Ala Leu His Asp
Pro Asn Leu Asn Glu Glu Gln Arg Asn Ala Arg Ile Arg Ser Ile Arg
Asp Asp Cys (SEQ ID NO: 22),
 
Phe Asn Met Gln Gln Gln Arg Arg Phe Tyr Glu Ala Leu His Asp
Pro Asn Leu Asn Glu Glu Gln Arg Asn Ala Arg Ile Arg Ser Ile Arg
Asp Asp (SEQ ID NO: 23); and
 and

Gly Lys Asn Met Gln Cys Gln Arg Arg Phe Tyr Glu Ala Leu His
Asp Pro Asn Leu Asn Glu Glu Gln Arg Asn Ala Arg Ile Arg Ser Ile
Arg Asp Asp Cys (SEQ ID NO: 24).

18. The method according to claim 17, wherein
the IgG binding peptide is: 
Gly Pro Asp Cys Ala Tyr His Arg Gly Glu Leu Val Trp Cys Thr
Phe His (SEQ ID NO: 25);
 
Gly Pro Asp Cys Ala Tyr His Lys Gly Glu Leu Val Trp Cys Thr
Phe His (SEQ ID NO: 26);
 
Gly Pro Asp Cys Ala Trp His Arg Gly Glu Leu Val Trp Cys Thr
Phe His (SEQ ID NO: 39); or
 or

Gly Pro Asp Cys Ala Trp His Leu Gly Glu Leu Val Trp Cys Thr
Phe His (SEQ ID NO: 40).

19. The method according to any one of claims 12 to 18, wherein
the IgG is an antibody against a target substance characteristic to immune cells which delivers the peptide vaccine

20. The method according to claim 19, wherein
the immune cells are selected from the group consisting of antigen-presenting cells, dendritic cells, and B cells.

21. The method according to 19 or 20, wherein
the IgG is selected from the group consisting of an anti-CD40 antibody, an anti-PD-L1 antibody, an anti-DEC205 antibody, an anti-DCIR antibody, an anti-Mannose receptor antibody, an anti-DC-SIGN antibody, an anti-CD11c antibody, and an anti-Dectin-1 antibody.
